(19) Europäisches Patentamt
European Patent Office
Office européen des brevets

(11) Publication number: **0 171 005 B1**

(12) **EUROPEAN PATENT SPECIFICATION**

(45) Date of publication of patent specification: **23.11.89**

(51) Int. Cl.$^4$: **C 07 D 473/08,** C 07 D 473/10, A 61 K 31/52

(21) Application number: **85109446.6**

(22) Date of filing: **26.07.85**

(54) **Xanthinylmethylthiazolidines, process for their preparation and pharmaceutical compositions containing them.**

(30) Priority: **27.07.84 IT 2208884**
**27.06.85 IT 2132385**

(43) Date of publication of application:
**12.02.86 Bulletin 86/07**

(45) Publication of the grant of the patent:
**23.11.89 Bulletin 89/47**

(84) Designated Contracting States:
**AT BE CH DE FR GB IT LI LU NL SE**

(56) References cited:
**FR-A-1 523 273**
**FR-A-2 480 286**
**GB-A-1 509 409**

(73) Proprietor: **BOEHRINGER BIOCHEMIA ROBIN S.p.A.**
**Via S. Uguzzone, 5**
**I-20126 Milan (IT)**

(72) Inventor: **Spinelli, Silvano**
**Via S. Uguzzone, 5**
**I-20126 Milan (IT)**
Inventor: **Russo, Raimondo**
**Via S. Uguzzone, 5**
**I-20126 Milan (IT)**
Inventor: **Tofanetti, Odoardo**
**Via S. Uguzzone, 5**
**I-20126 Milan (IT)**
Inventor: **Tognella, Sergio**
**Via S. Uguzzone, 5**
**I-20126 Milan (IT)**

(74) Representative: **Bianchetti, Giuseppe**
**Studio Consulenza Brevettuale Via Rossini, 8**
**I-20122 Milan (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 171 005 B1

**Description**

The present invention relates to antitussive, mucus regulating, and bronchodilators xanthinylmethyl-thiazolidines, to a process for their preparation and to pharmaceutical compositions containing them.

The compounds of the invention are xanthinylmethyl-thiazolidines of formula I

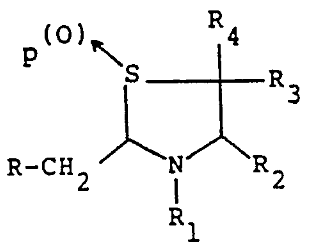

(I)

wherein

R is 1,3-dimethylxanthin-7-yl or 3,7-dimethylxanthin-1-yl;

$R_2$ is hydrogen, a carboxy group or, when p = 0 and $R_1 = R_3 = R_4 = H$, a carboxy group esterified with $C_1$—$C_5$ alkohols;

both $R_3$ and $R_4$ are hydrogen or methyl;

p is zero or 1;

$R_1$ is: hydrogen, a $C_1$—$C_2$ alkyl sulphonyl group, an unsubstituted or mono or poly-substituted phenyl sulphonyl group, or an acyl group of formula

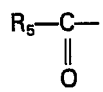

wherein $R_5$ is:

hydrogen, $-(CH_2)_n-Q$ and 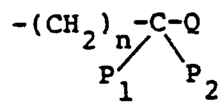

wherein n is 0 or an integer from 1:7; $P_1$, $P_2$, are both hydrogen or one of them is hydrogen and the other one is lower $C_1$—$C_4$ alkyl or phenyl and Q is selected in the group consisting of: a hydrogen; $C_3$—$C_4$-branched alkyl; a $C_3$—$C_7$ cycloalkyl; free carboxy or $COOC_2H_5$ when $R_2 = R_3 = R_4 = H$; an halogen atom; SH; —$NH_2$; a mono or di-substituted amino, t-butoxy carbonylamino or $C_1$—$C_2$ acylamino group; an ether —O—T or thioether S—T chain, wherein T is an unsubstituted or mono- or polysubstituted phenyl ring or a group of formula $(CH_2)_m$—$T_1$, wherein $T_1$ is selected in the group consisting of H, OH, $OCH_3$, $OC_2H_5$, $HOCH_2$—$CH_2$, free carboxy group or $COOC_2H_5$ when $R_2 = R_3 = R_4 = H$, $NH_2$, a $C_1$—$C_2$-acylamino or mono- or disubstituted amino group, or a group of formula

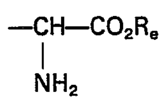

wherein $R_e$ is hydrogen, methyl or ethyl, and m is an integer from 1 to 3; a phenyl, phenoxy or phenylthio ring unsubstituted or mono- or polysubstituted in the m, o, and p-positions;

a group of formula —$(CH_2)_m$—SCO—$(CH_2)_nP_3$ wherein m and n have the above defined meanings and $P_3$ is a lower $C_1$—$C_7$, linear or branched alkyl chain, a $C_3$—$C_6$-cycloalkyl, a disubstituted amino group, a phenyl or phenoxy ring, optionally mono- or polysubstituted in the o, m and p-positions; an alkenyl chain of formula

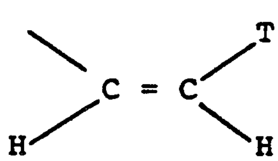

wherein T, in addition to the above defined meanings, may also be hydrogen.

The term "mono substituted amino group" comprises within the meanings thereof, an amino group substituted by a $C_1$—$C_6$, linear or branched alkyl group or by groups having formula

$$-CH_2-CH_2-O-CH_2-CH_3, \quad -CH_2-CH_2-O-CH_2-CH_2-OH,$$

$-CH_2-CH_2-NH-CH_2-CH_3$, $-CH_2-CH_2-NH-CH_2-CH_2-OH$ or

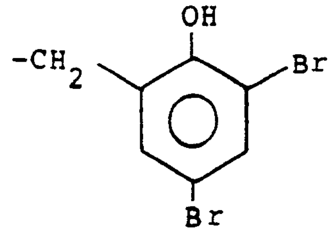

The substitutents of a disubstituted amino group according to the invention may be linear or branched $C_1$—$C_6$ alkyl groups or, taken together, they represent an unsaturated or saturated nitrogen ring such as morpholin-1-yl, pyrrolidin-1-yl, piperidin-1-yl, 4-methyl-piperazin-1-yl, thiomorpholin-1-yl, 4-ethyl-piperazin-1-yl, 4-(2'-fluorophenyl)piperazin-1-yl; imidazol-1-yl, 3-pyridyl, 4-pyridyl.

Finally, the term "mono- or polysubstituted phenyl", according to the invention, means phenyl groups which are substituted by a fluorine atom in the para position, by chlorine atoms in the meta and/or para positions or by a $CF_3$ in the meta positions, or a phenyl group of formula

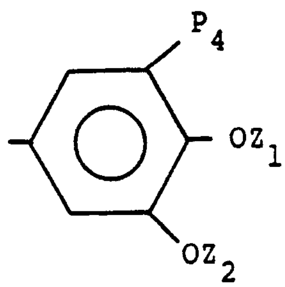

wherein $Z_1$ is H or $COCH_3$ and $Z_2$ is H, $CH_3$ or $COCH_3$ and $P_4$ is selected in the group consisting of hydrogen, aminomethyl, $C_1$—$C_2$-acylaminomethyl or mono- or di-substituted aminomethyl group, as above defined.

Typical examples of a saturated or unsaturated heterocylic ring containing one or more heteroatoms such as O, S, N, are: morpholin-1-yl, pyrrolid-1-yl, piperidin-1-yl, 4-methylpiperazin-1-yl, thiomorpholin-1-yl, 4-ethyl-piperazin-1-yl, 4-(2'-hydroxyethyl)piperazin-1-yl, 4-(4'-fluorophenyl)piperazin-1-yl, imidazol-1-yl, 3-pyridyl, 4-pyridyl.

Typical examples of pharmacologically acceptable non-toxic bases are organic bases e.g. organic amines such as methylamine, dimethylamine, trimethylamine, ethylamine diisopropylamine, N-methyl-N-hexylamine, tromethamine, cyclohexylamine, N-methyl-N-cyclohexylamine, α-phenylethylamine, β-phenylethylamine, N,N-dimethylethanolamine, N,N-diethylethanolamine, ethylenediamine, piperidine, morpholine, piperazine, galactamine, N-methylglucamine, ephedrine, lysine, arginine; and inorganic bases such as alkali and alkali-earth metal hydroxydes.

Typical examples of pharmacologically acceptable, non-toxic acids are organic acids such as acetic, formic, propionic, fumaric, maleic, malic, malonic, benzoic, salicyclic, 3,4,5-trimethoxybenzoic, methanesulphonic, benzenesulphonic, canfosulphonic, lactic, aspartic, glutamic, L- and D-2-phenyl-thiazolidin-5-carboxylic acid, cystin and cystein; and inorganic acids such as nitric, phosphoric, sulphoric, hydrochloric, hydrobromic acid.

Preferred salts of the invention comprise compounds of formula I wherein $R_2$ is a carboxy group salified with one of the above cited bases. Salts of piperazine and imidazole derivatives are even more preferred.

In the formula of this specification the wavy line bond

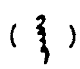

indicates that the substituents has not a definite stereochemical identity, i.e., that the substituent may be both of (R) and (S) configuration; the broke line

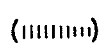

indicates that a substituent is of (S) absolute stereochemistry; the heavy solid line

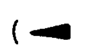

indicates that a substituent is of (R) absolute configuration.

The compounds of the invention of formula I, wherein $R_2$, $R_3$ and $R_4$ are hydrogen and $R_1$ is an acyl group, are particularly preferred as mucus regulating agents.

The compound of formula I, wherein $R_2$, $R_3$ and $R_4$ are hydrogen and $R_1$ is a substituted or unsubstituted cinnamoyl group, are particularly preferred as bronchodilatory agents.

Specific examples of the preferred compounds of the invention are the following:

2-[(1',3'-dimethylxanthin-7'-yl)methyl]thiazolidin-4-carboxylic acid and their methyl and tert-butyl esters;

2-[(3',7'-dimethylxanthin-1'-yl)methyl]thiazolidin-4-carboxylic acid and their methyl, ethyl and tert-butyl esters;

2-[(1',3'-dimethylxanthin-7'-yl)methyl]thiazolidine;

2-[(3',7'-dimethylxanthin-1'-yl)methyl]thiazolidine;

2-[(1',3'-dimethylxanthin-7'-yl)methyl]-3-(imidazol-1'-yl)acetyl/thiazolidine;

2-[(3',7'-dimethylxanthin-1'-yl)methyl]-3-(imidazol-1'-yl)acetyl/thiazolidine;

2-[(1',3'-dimethylxanthin-7'-yl)methyl]-3-[3',4'-hydroxy)cinnamoyl]thiazolidine;

2-[(3',7'-dimethylxanthin-1'-yl)methyl]-3-[3',4'-dihydroxy)cinnamoyl]thiazolidine;

2-[(1',3'-dimethylxanthin-7'-yl)methyl]-3-(3'-methoxy-4'-hydroxy)cinnamoyl]thiazolidine;

2-[(3',7'-dimethylxanthin-1'-yl)methyl]-3-(3'-methoxy-4'-hydroxy)cinnamoyl]thiazolidine;

2-[(3',7'-dimethylxanthin-1'-yl)methyl]-3-(3'-[3'-(4''-hydroxy)cinnamoyl]thiazolidine;

2-[(1',3'-dimethylxanthin-7'-yl)methyl]-3-(3'-morpholino-methyl-4'-hydroxy-3'-methoxy-cinnamoyl)-thiazolidine;

2-[(1',3'-dimethylxanthin-7'-yl)methyl]-3-(3'-pyrrolidyl-methyl-4'-hydroxy-3'-methoxy-cinnamoyl]-thiazolidine;

2-[(3',7'-dimethylxanthin-1'-yl)methyl]-3-(3'-pyrrolidinylmethyl-4'-hydroxy-3'-methoxy-cinnamoyl)-thiazolidine;

2-[(1',3'-dimethylxanthin-7'-yl)methyl]-3-(3'-thia-5'-carboxy-5'-acetylamino-pentanoyl]thiazolidine;

2-[(1',3'-dimethylxanthin-7'-yl)methyl]-3-[4'-thia-6'-carboxy-6'-acetyl-amino-hexanoyl]thiazolidine;

2-[(1',3'-dimethylxanthin-7'-yl)methyl]-3-[3'-(2''-ethoxyethyloxy)acetyl]thiazolidine;

2-[(1',3'-dimethylxanthin-7'-yl)methyl]-3-[3'-(imidazol-1'-yl)propionyl]thiazolidine;

2-[(1',3'-dimethylxanthin-7'-yl)methyl]-3-glycinyl-thiazolidine;

2-[(3',7'-dimethylxanthin-1'-yl)methyl]-3-glycinyl-thiazolidine;

2-[(1',3'-dimethylxanthin-7'-yl)methyl]-3-(n-acetyl)glycinyl-thiazolidine;

2-[(1',3'-dimethylxanthin-7'-yl)methyl]-3-[3'-(2''-hydroxyethylamino)ethnylamino-propionyl]-thiazolidine;

2-[(1',3'-dimethylxanthin-7'-yl)methyl]-3-[3'-(2''-hydroxyethylamino)ethylamino-propionyl]-thiazolidine;

2-[(1',3'-dimethylxanthin-7'-yl)methyl]-3-[3'-(4''-hydroxyethylpiperazin-1'yl)proionyl]thiazolidine;

The compounds of the invention are prepared by reacting compounds of formula II

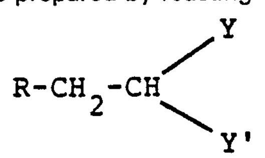

(II)

wherein R is as above defined and both Y and Y' are lower $C_1$—$C_2$ alkoxy or, taken together, form a carbonyl group, with an amine alkanethiol of formula III

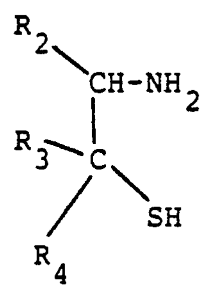

(III)

wherein $R_2$, $R_3$ and $R_4$ are as above defined, to give a compound of formula (Ia)

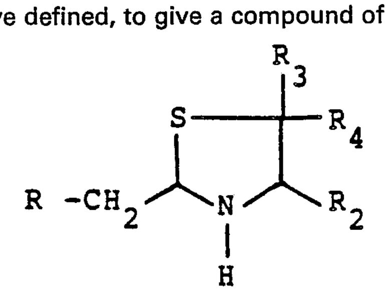

(Ia)

wherein R, $R_2$, $R_3$, $R_4$ are as above defined.

Compounds of formula I wherein $R_2$ is hydrogen may be optionally subjected to optical resolution; in the case where $R_2$ is a free or an esterified carboxy group the single diasteroisomers and/or racemic mixtures of the diastereoisomers can be optionally obtained.

Thiazolidines of formula Ia and enantiomers or diastereoisomers thereof can be optionally subjected to subsequent acylation by reaction with an acylating agent selected in the group consisting of: a cyclic anhydride such as succinic and glutaric anhydride; an activated species of a carboxylic acid of the formula IVa and IVb; and a sulphonyl halide of the formula IVc:

$$Q-(CH_2)_n-COZ$$

(IVa)

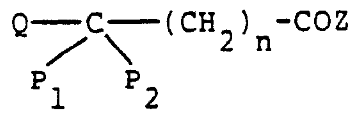

(IVb)

$$P_5-SO_2-Hal$$

(IVc)

wherein:

Q, $P_1$, $P_2$ and n are as above defined;

$P_5$ is a $C_1$—$C_2$-alkyl group or an unsubstituted or mono or polysubstituted phenyl ring;

Hal is chlorine, bromine and iodine;

Z is a recognized species able to activate a carboxy group such as chlorine, bromine, azide, —O—CO—D where D is a $C_1$—$C_5$ lower alkoxy or benzyloxy, a $C_1$—$C_5$ lower alkyl (mixed anhydride and anhydride) and activated esters.

The obtained acylated thiazolidines have the formula Ib

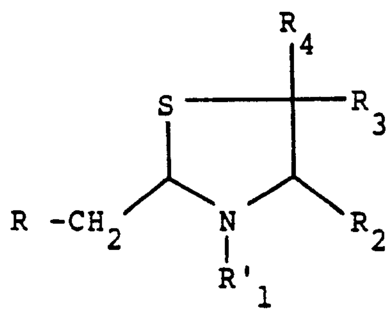

(Ib)

wherein R, $R_3$, $R_4$ and $R_2$ have the above mentioned meanings and $R'_1$ is a group of formula $SO_2$—$P_5$ or CO—$R_5$ wherein $P_5$ and $R_5$ are as above defined.

Using in the acylation reaction a carbodiimide as activating species of the carboxy group, the acylation reaction can be optionally performed by reaction of a thiazolidine of formula I with an acid of the formulae IVa, IVb wherein Z is hydroxy.

Compounds Ib can be optionally oxidized by a suitable reagent to compounds of formula Ic

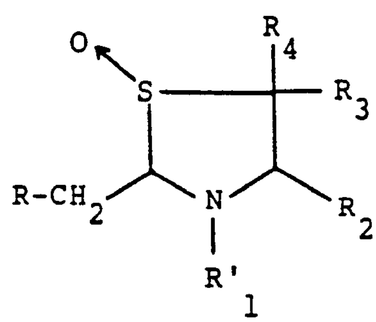

(Ic)

wherein the substituents have the above defined meanings.

Compounds of the general formula Id

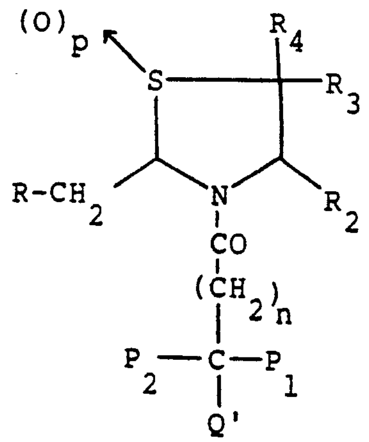

(Id)

wherein R, $R_2$, $R_3$, $R_4$, p are as above defined and at least one of $P_1$ and $P_2$ is hydrogen, and the other one is hydrogen, methyl or phenyl, Q′ is a halogen atom and n is preferably zero, may be optionally reacted with thiocarboxylic acid salts of the formula:

$$P_3—(CH_2)_n—CO—S^{(-)} \quad Base^{(+)}$$

wherein $P_3$ is as above defined and the base is selected in the group consisting of sodium, potassium, magnesium, calcium, lower trialkylammonium, phenyldialkylammonium, to give the compounds of formula Id in two steps, wherein Q′ is $P_3—(CH_2)_n—CO—S$; these latter compounds can be optionally reacted with ammonia, too, to give a compound of formula Id wherein Q′ is a free thiol group, from which compounds of formula Id, wherein Q′ is an alkylthioether group, can be optionally obtained by reaction with an alkyl halide in the presence of a base.

Finally the compounds of formula Id wherein n is zero, $P_1$ and $P_2$ are hydrogen and Q′ is Cl, Br or I may be reacted with triphenylphosphine in an inert solvent (benzene, acetonitrile, THF, etc.), to give the corresponding phosphonium salts of formula Id, where Q is

$$\overset{(+)}{P}(C_6H_5)_3X^-$$

which are optionally converted into a stabilized ylide compound of formula Ie

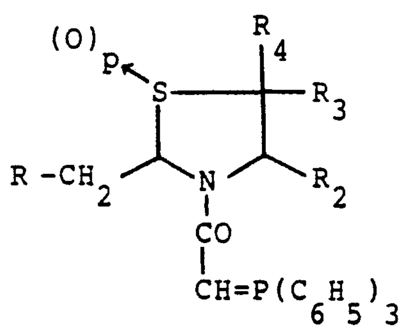

(Ie)

wherein R, $R_3$, $R_4$, $R_2$ and p are as above defined, which are then optionally reacted with an aldehyde of the formula V

$$T—CHO \qquad (V)$$

wherein T is also as above defined to give, after removal of the protective groups, 3-thiazolidine acryl amides of the formula If

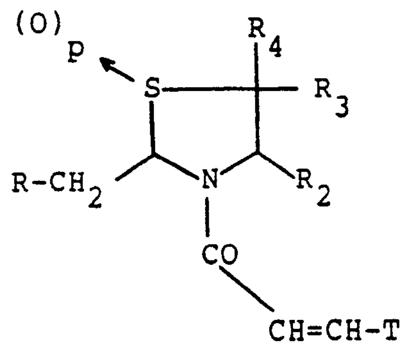

(If)

which, when T is H, $C_1$—$C_4$-lower alkyl or phenyl, can be optionally reacted with nucleophiles such as amines i.e. $H_2N$—$(CH_2)_m$—$T_1$, monosubstituted or disubstituted amines or thiols (i.e.: HS—$(CH_2)_m$—$T_1$, unsubstituted and mono or polysubstituted thiophenols) to obtain a Michael adduct of formula (Ig)

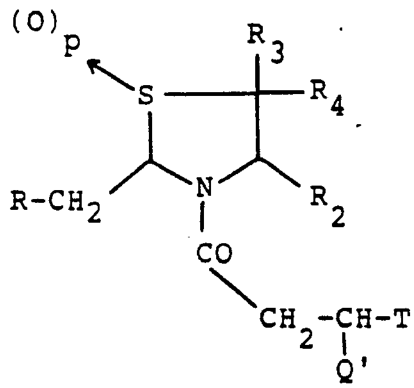

(Ig)

wherein R, $R_2$, $R_3$, $R_4$, T and p are as above defined and Q' is selected in the group consisting of HN—$(CH_2)_mT_1$, mono and disubstituted amino groups, S—$(CH_2)_nT_1$ (m, n, $T_1$ being as above defined) unsubstituted, mono or polysubstituted phenylthio group.

The cyclization of a compound of formula II with an aminoalkanethiol of formula III to give a 2-substituted thiazolidine ring may be performed by reaction with either a stoichiometric amount of a small excess of the amino alkanethiol in an aqueous solvent, either in the presence or absence of a catalytic amount of its ammonium salt such as acetate, formiate, camphorsulfonate, hydrochloride.

Suitable solvents are, for example, water, methanol, ethanol, acetic acid and mixtures thereof.

The reaction is preferably carried out at temperatures ranging from about −20°C to the solvent's reflux temperature; preferably the reaction is performed at room temperature.

The reaction times range from few minutes to several days, but usually they do not exceed two hours and often a few minutes are sufficient to complete the reaction.

The optical resolution of the compounds of formula Ia may be optionally carried out by salification with an optically active acid such as for example d- and 1-camphor sulfonic acid, d- and l-mandelic acid, d- and 1-6-exo-chloro-7-syncarboxy-bicyclo[2,2,1]heptan-3-one-3,3-ethylendioxy followed by crystallization till constant rotatory power and recovery of the optically active 2-subsituted-thiazolidine.

Suitable solvents are, for example, alcohols such as methanol, ethanol, 1-propanol, 2-propanol, ethers such as ethylether, isopropylether, dioxane, tetrahydrofuran, ester such as ethylformiate and ethylacetate, and hydrocarbons such as benzene, toluene, cyclohexane, hexane and mixtures thereof.

The optical resolution is preferably carried out at room temperature and few crystallisations are generally necessary to obtain a constant rotatory power.

The acylation of the compounds of formula Ia with an acylating agent of formula IV may be performed by reaction with either a stoichiometric amount or a small excess of acylating agent in an inert solvent in the presence of either a stoichiometric amount or of an excess of a base. Suitable solvents are, for example, halogenated hydrocarbons, such as $CH_2Cl_2$, $CHCl_3$, Cl—$CH_2CH_2Cl$; ketones such as acetone and butan-2-one; hydrocarbons such as hexane, cyclohexane, benzene, toluene, piridine and mixtures thereof.

A nearly stoichiometric amount of the base for any molecule of the acylating agent is useful. Such a base may be an inorganic base e.g. an alkali or an alkali-earth metal oxide, carbonate or bicarbonate e.g. CaO, $CaCO_3$, $K_2CO_3$, $Na_2CO_3$, $NaHCO_3$; an organic base such as a tertiary amine, e.g. trimethylamine, tributylamine; or an aromatic base, e.g. pirdine, an alkyl substituted piridine, a N,N-dialkylaniline, or an anionic ion exchange resin.

The acylation reaction is preferably carried out at temperature ranging from about −40°C to about the solvent's reflux temperature; preferably the reaction is carried out at room temperature.

Using in the acylation reaction a carboxylic acid of formula IVa and IVb (Z = OH), the reaction is performed in presence of an excess of a condensing agent, such as a carbodiimide solvent, at room temperature, optionally in the presence of 4-dimethylamino piridine as a catalyst.

The oxidation of the compounds of formula Ib to obtain a thiazolidine sulphoxide of formula Ic may be performed by reaction with either a stoichiometric amount or small excess or organic peracid such as monoperphatlic acid, m-chloroperbenzoic, peracetic acid, performic acid and perbenzoic acid in an inert solvent either in presence or in the absence of a base.

Preferred oxidizing agenty is a stoichiometric amount of monoperphtalic aicd, the preferred solvent is ethyl acetate and the reaction is preferably performed in presence of an excess of $NaHCO_3$.

The oxidation reaction is preferably carried out at temperatures ranging from about −25°C to room temperature; preferably the reaction is performed at 0°C.

The thiolation of a compound of formula Id, wherein Q' is a halogen atom, may be carried out by treatment with either a stoichiometric amount or an excess of a salt of a thiocarboxylic acid such as for example, sodium, potassium salt and/or "in situ" formed salt of the thiocarboxylic acid with an organic base such as trimethylamine, triethylamine, tributylamine, in an inert solvent.

Suitable solvents are, for example, halogenated hydrocarbons, ketones, esters, ethers, alcohols and the mixtures thereof.

The reaction is preferably carried out at room temperature and the reaction times range from few minutes to several hours but, usually, the reaction times do not exceed two hours at room temperature.

A free thiol group may be obtained by reaction of the thioaycl compounds with an excess of aqueous ammonia solution in inert gas atmosphere.

The reaction is performed at room temperature, suitable solvents are alcohols such as methanol, ethanol, glycols and ethers miscible with aqueous ammonia solutions such as dimethoxyethane, dioxane, tetrahydrofuran and mixtures thereof.

The acylation of the free thiol group may be performed as above described.

The alkylation of the free thiol group may be performed by treatment of the potassium and/or sodium salt of the thiol compound with an alkyl halide.

The reaction between the stabilized ylides of the formula Ie and the aldehydes of formula V is the well-known Wittig reaction whose experimental procedure is also well-known to the people skilled in the art; generally it is performed by mixing the reagents in equimolecular ratio in an inert solvent such as an halogenated solvent, an ether solvent (THF, dimethoxyethane), acetonitrile, cyclohexane, benzene, toluene, hexane, dimethylsulphoxide or using a mixture thereof, preferably at room temperature.

The Michael addition of the above defined nucleophiles to the acrylamides of the formula If is also a well-known reaction. Preferred solvents are alcohols and the reaction is made mixing the reagents and heating at the reflux temperature.

The compounds of formula II are known compounds or may be prepared by known methods (De Martiss et al., "Il Farmaco", Ed. Sc., *9*, 526, *1956)*.

Particularly, the compounds of formula II wherein Y and Y' are methoxy or ethoxy are prepared by reacting an appropriate α-halogenated acetaldehyde-acetal with an alkali salt (such as lithium, sodium or potassium) of 1,3-dimethylxanthine or 3,7-dimethylxanthine in a suitable solvent such as dimethylformamide, dimethylsulphoxide or water and mixures thereof.

The compounds of the invention are therapeutically active substances, devoid of acute, subacute or chronic toxic effects, suitable for use as antitussive agents, mucus regulating agents or bronchodilatatory agents.

In fact, for example, the compounds of the invention never exhibit acute toxic effects in mice and rats.

$LD_{50}$ values above 1 g/kg are generally determined both after oral and intraperitoneal administration of the compounds of the invention.

The compound of the invention: 2-[(1',3'-dimethylxanthin-7'-yl)methyl]thiazolidine differs from the other ones because it shows in mice a $LD_{50}$ of 0.6 g/kg (ops) and 0.48 g/kg (i.p.).

The techniques described by Charlier et al (Arch. Int. Pharmacodyn. *134*, 306, 1961) and by Stefko et al. (J. Pharm. Exp. Therap., *108*, 217, 1953), adapted with minor modifications, are used to investigate the antitussive properties of the compounds of the invention. Codeine phosphate is used as positive reference compound.

According to the Charlier procedure, Guinea pigs are exposed to a citric acid aerosol (7.5%) and cough is recorded 60 minutes after the antitussive treatment by i.p. route. Male animals of 300—400 g body weight are used in all the experiments. Guinea pigs are placed in a perspex box (20 × 30 × 30 cm) connected to an aerosol compressor; the animals are subjected to a critic acid saturated atmosphere, 60 minutes after the i.p. injection of a compound of the invention (0.1 and 0.3 M solutions; 1 ml/kg).

The total number of cough strokes and the delay of the first cough are recorded during the first five minutes.

Five guinea pigs are used for each dose level and the results are compared with controls (vehicle treatment) and codeine phosphate 0.07 M solution (standard treatment).

$ED_{50}$ and the corresponding delay of first cough stroke are calculated.

The data obtained by i.p. administration are confirmed by oral administration.

In the Stefko procedure, conscious mongrel dogs (15 ± 3 kg body weight) are used; the compounds of

tl ' invention are tested for their ability to inhibit cough induced by electric stimulation.

Under general anesthesia, two insulated nichrome wires (0.4 mm diameter) are surgically implanted into the submucosa of the trachea.

About two days later, the dogs are placed in a sling and the exteriorized electrodes are connected to an electric stimulating device (such as Grass S 48 Mod.).

The stimulation parameters such as : delay 0.01 msec, duration 1 msec, frequency 30 Hz, are used.

An electrical stimulus of 1 sec is applied 10 times at 5 sec intervals in order to determine the minimum voltage necessary for eliciting reproducible cough responses. Then, tussive responses to the electrical stimuli are registered at 15, 30, 60, 90, 120, 150, 180, 240, 300 and 360 minutes after oral administration of the compounds of the invention.

The tussive responses are scored as follows:

1: no response;
2: sigh or exaggerated expiration;
3: marked cough
4: one marked cough and one exaggerated expiration;
5: two marked coughs.

Six dogs are employed for each determination and the man activity is calculated.

The inhibition percent of the cough response and the duration of activity are compared with codeine.

In the guinea pig cough test, codeine phosphate shows and $ED_{50} \simeq 23$ mg/kg with a 170% delay.

In the dog cough test, codeine phosphate when tested at 8 mg/kg shows a 52% inhibition of the cough stimulus for a long time (about 2—4 hours).

The mucus regulating properties of the compounds of the invention are investigated "in vivo" by means of experiments carried out on male New Zealand white rabbits. The animals are submitted to inhalation of a 5% $H_2SO_3$ aqueous solution by aerosol (exposition of 3 hours a day for 3 alternate days), to induce a chronic bronchial inflammatory disease which causes a production of pathological sputum in the bronchial tree.

Aim of the experiments is to evaluate modifications of selective parameters such as dry weight of the sputum, viscosity, content in proteins, phospholipids, galactose, sialic acid and fucose, induced in this pathological sputum by pharmacological treatment with the compounds of the invention.

To this purpose, the sputum produced in the bronchial tree is collected daily, through a "T" shaped glass cannula, inserted in the trachea, before and after oral twice a day (9.00 a.m. and 4.00 p.m.) treatment with one of the compounds of the invention.

The schedule treatment procedure is shown in the following Scheme 1:

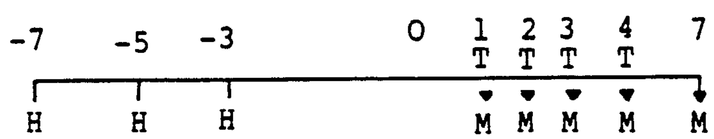


Scheme I

where 0 indicates the day of implication of the tracheal cannula; -7-5-3 indicate the days of $H_2SO_3$ treatment (H); 1, 2, 3, 4 indicate the days of oral aministration of the compounds of the invention (T) indicates the day of the animal sacrifice after such mucus collection.

The sputum samples are stored in the freezer (−20°C) until the investigation of biological and rheological parameters is carried out.

The samples are investigated for relative viscosity, measured at 37°C with a Brookfield viscometer (model LT VD) equipped with a 1,565° cone-plate, and aliquoted for biochemical analysis and fucose contents. Dry weight of the mucus is also determined. For each parameter, cumulative data are referred to a seven day period after the beginning of the treatment and they are extrapolated using the AUC method (area under curve) calculated by the trapezoidal rule. Finally, the data are compared with data obtained from controls (vehicle treatment), non bronchitic rabbits (vehicle treatment) and positive controls bronchitis rabbits treated with 0.153 m 2-carboxymethyl cysteine.

In Table 1 the AUC values, determined for the various parameters, after the treatment of bronchitic rabbits with 2-carboxymethyl cysteine are reported.

<u>TABLE 1</u> - AUC as a % compared to bronchitis controls

| Treatment | Viscosity (cps) | Mucus production ml/kg | Protein mg/ml | Phospholipids mg/ml | Galactose mg/ml | Sialic acid mg/ml | Fucose mg/ml |
|---|---|---|---|---|---|---|---|
| Controls | 100 | 100 | 100 | 100 | 100 | 100 | 100 |
| S-carboxy-methyl cy-steine 0.153 M | 52 | 97 | 105 | 108 | 64 | 60 | 56 |

As it is evident, all the parameters are influenced by the pharmacological treatment with S-carboxymethyl cysteine.

An ideal mucoregulating agent should reduce the viscosity and the protein content of the mucus.

In pathological cases, the high protein content of the mucus could be related to an abnormal production of the mucus macromolecules and also to an abnormal passive transport of seric proteins through the capillary vessels.

At the same time, the reduction of the mucoprotein content should be associated with a reduction of the contents of galactose and sialic acid in the sputum, so indicating a lower tendency to produce mucines which contribute to the viscosity of the mucus.

Fucose content is related to the production of neutral mucines.

The increase in the mucus volume is connected to a reparative local process for which a liquefation of the mucus is obtained. This event is particularly desirable when, as in some pathological conditions, the mucus of the patients appears to be adhesive of the respiratory ways.

The results obtained in two compounds of the invention, administered orally in equimolecular amounts in comparison with the standard S-carboxymethyl cysteine, are shown in Table 2.

TABLE 2

| Treatment | AUC as a 100% compared to bronchitic controls | | | | | | |
|---|---|---|---|---|---|---|---|
| | Viscosity (cps) | Mucus produc-tion (ml/kg) | Protein (mg/ml) | Phospho-lipids (mg/ml) | Galactose (mg/ml) | Sialic acid (mg/ml) | Fucose (mg/ml) |
| Animal untreated with $H_2SO_3$ | 90 | 72 | 96 | 79 | 30 | 78 | 85 |
| Bronchitic animal (5% $H_2SO_3$ aerosol) | 100 | 100 | 100 | 100 | 100 | 100 | 100 |
| 2-[(1',3'-Dimethylxanthin-7'-yl)methyl]-4-carboxy-thiazolidine | 55 | 128 | 80 | 78 | 82 | 81 | 77 |
| 2-[(1',3'-Dimethylxanthin-7'-yl)methyl]-thiazolidine | 16 | 80 | 58 | 30 | 5 | 101 | 66 |

The 2-[(1,3-dimethylxanthin-7'-yl)methyl]thiazolidine-4-carboxylic acid is a typical compound of the invention, having mucus regulating properties as well as good antitussive activity; in fact the compound shows and $DE_{50}$ of 62 mg/kg (213% delay) in the guinea pig cough test, exhibiting also a more favourable duration of action in comparison to codeine. At does of 20 mg/kg the compound shows a 32% inhibition of the cough stimulus in dogs and favourably compares with codeine for its duration of action. The 4-carboxy-xanthinyl-methylthioazolidines having the carboxy group free or esterified are also effective as antitussive agents with $ED_{50}$'s ranging from 50 to 65 mg/kg in the Guinea pig cough test and are also able to induce inhibition from 38% and 50% in the dog cough stimulus test.

This activity as antitussive agents is also combined with mucus regulating activity which is at least equipotent with S-carboxymethyl-cysteine.

The guinea pig cough test and the dog cough test are effective and reliable testing prodecures for the screning of substances in the treatment of cough of different origines and in the relief of the pain induced by tussive attacks.

Accordingly, the compounds of the invention may be useful in the treatment of patients in order to reduce and to prevent tussive attacks. Therefore the compounds of the invention are also useful to inhibit cough paroxysm which could develop syncope.

Prevention and inhibition of strenuous coughing is also highly useful because cough may produce rupture of an emphysematous bled and rib fractures.

Although cough induced rib fractures may occur in otherwise normal patients, their occurrence should at least raise the possibility of pathologic fractures, which are seen in multiple myeloma, osteoporosis and osteolytic metastase.

Particularly preferred compounds of the invention as antitussive agents are:

2-[(1',3'-dimethylxanthin-7'-yl)methyl]thiazolidine-4-carboxylic acid, and their esters with $C_1$—$C_5$ alcohols;

2-[(3',7'-dimethylxanthin-1'-yl)methyl]thiazolidine-4-carboxylic acid and their esters with $C_1$—$C_5$ alcohols;

2-[(1',3'-dimethylxanthin-7'-yl)methyl]-3-[3'-(2''-hydroxyethyl-aminoethylamino)propionyl]-thiazolidine;

2-[(1',3'-dimethylxanthin-7'-yl)methyl]-3-[3'-(4''-hydroxyethyl-piperazin-1''-yl)propionyl]thiazolidine.

A moderate activity as antitussive agent combined with a very high mucus regulating activity is highly desirable in some pahtological conditions such as chronic bronchitis, when it is not always desirable to suppress a cough productive of significant quantities of sputum. In some cases, an early suppression of the cough could involve retention of the mucus in the tracheobronchial tree, negative interference with the distribution of the alveolar aeration and with the ability of the lung to resist infections.

As a consequence the changes induced in the mucus by the treatment with selective mucus regulators may reduce the number of cough attacks.

In accordance with this aim, particularly useful and preferred substances which exhibit a high mucus regulating activity together with a reduced antitussive potency are:

2-[(1',3'-dimethylxanthin-7'-yl)methyl]thiazolidine;

2-[(3',7'-dimethylxanthin-1'-yl)methyl]thiazolidine and their 3-acetylthioacetyl derivatives;

2-[(1',3'-dimethylxanthin-7'-yl)methyl]-3-[3'-thia-5'-carboxy-5-acetylamino-hexanoyl]thiazolidine;

2-[(1',3'-dimethylxanthin-7'-yl)methyl]-3-[3'-(imidazol-1''-yl)propionyl]thiazolidine.

Particularly preferred mucus regulating agents are the amides of the 2-[(1',3'-dimethylxanthin-7'-yl)-methyl]thiazolidine with the acids: imidazol-1'-yl-alkanoic, caffeic, ferulic, (3-morpholinylmethyl-4-hydroxy-3-methoxy)cinnamic and (3-pyrrolidinylmethyl-4-hydroxy-3-methoxy)cinnamic.

Said compounds induce modifications of the evaluated rheologic parameters (protein, phospholipides, fucose, sialic acid, and viscosity) similar to those obtained with the starting thiazolidines but using lower dosage.

In particular these latter compounds of the invention are also characterized by pronounced ability to induce relaxation of the bronchial and tracheal smooth muscle. For instance the compound 2-[(1',3'-dimethylxanthin-7'-yl)methyl]-3-[3'-morpholinylmethyl-4'-hydroxy-5'-methoxycinnamoyl]thiazolidine is able to relax "in vitro" trachea smooth muscle strips of Guinea pig contracted by methacoline, with an $ED_{50}$ of $1{,}9 \cdot 10^{-4}$ M. The spasmolytic activity of the new substance favourably compared with that of dihydroxy-propyltheophylline ($ED_{50}$ $0.76 \cdot 10^{-4}$).

After intrajugular administration, the compound appears at least four times more active than aminophylline in the resolution of broncospasm induced in anhaestetized guinea pigs by i.v. hystamine (Konzett-Ressler test).

Compounds I are then effective antitussive, bronchodilatory and mucus regulating agents. They may be administered by oral, sublingual, intravenous, subcutaneous, intramuscular, rectal or inhalatory route.

The inhalatory route is particularly preferred when a mucus regulating action is required.

The preferred dose of the compounds of the invention will vary from 0.05 to about 5 mg/kg/day, depending on patient's conditions, weight and age, and administration route.

The preferred dose by inhalatory route will vary between 0.05 and 1 mg/kg/day.

As previously stated, the compounds of the invention can be administered either to humans or animals in a variety of forms, e.g., orally in the form of tablets, capsules or liquids; rectally in the form of

suppositories; parenterally, subcutaneously or intramuscularly, the intravenous administration being preferred in emergency situations; by inhalation in the form of aerosols or solutions for nebulizers; in the form of sterile implants for prolonged action. The pharmaceutical compositions containing the compounds of the invention may be prepared in conventional ways and contain conventional carriers and/or diluents.

For example, for intravenous injection or infusion, sterile aqueous isotonic solutions are preferred. For subcutaneous or intramuscular injection, sterile solutions or suspensions or non-aqueous media may be used; for tissue implants, a sterile tablet and silicone rubber capsule containing or impregnated with the compound is used.

Conventional carriers or diluents are, for example, water, gelatine, lactose, dextrose, saccharose, mannitol, sorbitol, cellulose, talc, stearic acid, calcium or magnesium stearate, glycol, starch, arabic gum, tragacanth gum, aliginic acid or alginates, lecithin, polysorbate, vegetable oils, etc.

For administration by nebulizer, a suspension or a solution of the compound of the invention, preferably in the form of a salt, such as the sodium salt in water (and/or the nitrate salt) can be used. Alternatively, the pharmaceutical preparation can be in the form of a suspension or of a solution of the compound of the invention in one of the usual liquified propellants, such as dichlorodifluoro-methane and dichlorotetrafluoroethane, administered from a pressurized container such as an aerosol bomb. When the compound is not soluble in the propellant it may be necessary to add a co-solvent, such as ethanol, dipropylene glycol and/or a surfactant to the pharmaceutical formulation.

The following examples illustrate but do not limit the present invention.

Example 1

An aqueous solution of cysteamine hydrochloride (1.65 g) and potassium acetate (1.47 g) in 10 ml of 70% aqueous acid is added to theophilline acetaldehyde dimethylacetale (2.4 g) and the mixture is heated for 3 hours at reflux temperature. The suspension is diluted with water, the precipitate is filtered, washed with water to give 2-[(1',3'-dimethylxanthin-7'-yl)methyl]thiazolidine (2.4 g), m.p. 141—143°C (from ethanol).

Example 2

A solution of theobromine acetaldehyde (2.4 g) in 70 ml of ethanol is added to a solution of cysteine hydrochloride (2.12 g) and potassium acetate (1.4 g) in 10 ml of water. The suspension is stirred overnight at room temperature, diluted with water and filtered. After crysallization from ethanol, 2.3 g of 2-[(3',7'-dimethylxanthin-1'-yl)methyl]thiazolidine-4-carboxylic acid, m.p. 157—160°C, are obtained.

Example 3

Using in the procedure of Example 1—2 cysteine ethylester hydrochloride, cysteine hydrochloride, d- and l-penicillamine and by reaction with theophilline acetaldehyde and theobromine acetaldehyde and with the corresponding dialkylacetals, the following compounds are prepared:

2-[(3',7'-dimethylxanthin-1'-yl)methyl]thiazolidine, m.p. 166—167°C;
2-[(1',3'-dimethylxanthin-7'-yl)methyl]thiazolidine-4-carboxylic acid, m.p. 147—150°C;
2-[(1',3'-dimethylxanthin-7'-yl)methyl]-4-carboethoxythiazolidine, m.p. 111—113°C;
2-[(3',7'-dimethylxanthin-1'-yl)methyl]-4-carboethoxythiazolidine, m.p. 129—130°C;
2-[(3',7'-dimethylxanthin-1'-yl)methyl]-5,5-dimethylthiazolidine-4-carboxylic acid, m.p. 132—135°C;
2-[(1',3'-dimethylxanthin-7'-yl)methyl]-5,5-dimethylthiazolidine-4-carboxylic acid, m.p. 125—130°C.

Example 4

Methanesulphonyl chloride (0.15 ml) is added, dropwise, to a stirred solution of 2-[(1',3'-dimethylxanthin-7'-yl)methyl]thiazolidine (0.25 g) in pyridine (3.5 ml), cooled at 0°C; after 30 minutes the mixture is heated at room tempeature and after one hour it is diluted with aqueous $H_2SO_4$ and extracted with $CHCl_3$.

After the usual work-up the residue is crystallized from ethanol to give 2-[(1',3'-dimethylxanthin-7'-yl)-methyl]-3-methanesulphonyl-thiazolidine (0.24 g), m.p. 206°C.

Example 5

Using in the procedure of example 4 ethanesulphonylchloride, benzenesulphonylchloride and p-toluensulphonylchloride, the following compounds are obtained:

2-[(1',3'-dimethylxanthin-7'-yl)methyl]-3-ethanesulphonylthiazolidine, m.p. 208°C;
2-[(1',3'-dimethylxanthin-7'-yl)methyl]-3-phenylsulphonylthiazolidine, m.p. 215—220°C;
2-[(1',3'-dimethylxanthin-7'-yl)methyl]-3-p-toluenesulphonylthiazolidine, m.p. 215—218°C;
2-[(3',7'-dimethylxanthin-1'-yl)methyl]-3-methanesulphonylthiazolidine, m.p. 210—213°C;
2-[(3',7'-dimethylxanthin-1'-yl)methyl]-3-ethanesulphonylthiazolidine, m.p. 210—258°C;
2-[(3',7'-dimethylxanthin-1'-yl)methyl]-3-methanesulphonylthiazolidine, m.p. 220—222°C;
2-[(3',7'-dimethylxanthin-1'-yl)methyl]-3-p-toluenesulphonylthiazolidine, m.p. 220—222°C;

Example 6

A solution of α-chloroacetyl chloride (0.31 ml) in 2 ml of dichloroethane, cooled at 0°C, is added dropwise to a stirred suspension of 2-[(1',3'-dimethylxanthin-7'-yl)methyl]thiazolidine (1 g) and 0.54 ml) of triethylamine in 10 ml of dichloroethane.

After 30 minutes at 0°C and 2 hours at room temperature, the mixture is partitioned between 50 ml of water and 50 ml of dichloroethane. The organic phase is washed with 5% aqueous $NaHCO_3$ and water, dried on sodium sulphate, and the solvent is evaporated under vacuum. The residue is crystallized from ethanol to give 1 g of 2-[(1′,3′-dimethylxanthin-7′-yl)methyl]thiazolidine, m.p. 205—207°C.

Example 7

According to the procedure of Example 6, and using 2-[(3′,7′-dimethylxanthin-1′-yl)methyl]-thiazolidine, 2-[(1′,3′-dimethylxanthin-7′-yl)methyl]thiazolidine, α-chloroacetyl chloride, α-bromopropionyl chloride, cyclopropylcarbonyl chloride, cycloexylpropionyl chloride, cyclopentylpropionyl chloride and ethyloxalyl chloride, the following compounds are obtained:

2-[(3′,7′-dimethylxanthin-1′-yl)methyl]-3-chloroacetylthiazolidine, m.p. 208—210°C;
2-[(3′,7′-dimethylxanthin-1′-yl)methyl]-3-cyclopropylcarbonyl-thiazolidine, m.p. 210—212°C;
2-[(3′,7′-dimethylxanthin-1′-yl)methyl]-3-(2′-bromo)propionyl-thiazolidine, m.p. 215—218°C;
2-[(3′,7′-dimethylxanthin-1′-yl)methyl]-3-(3′-cycloexyl)propionyl-thiazolidine, m.p. 170—172°C;
2-[(3′,7′-dimethylxanthin-1′-yl)methyl]-3-(3′-cyclopentyl)propionyl-thiazolidine, m.p. 175—177°C;
2-[(3′,7′-dimethylxanthin-1′-yl)methyl]-3-ethyloxalylthiazolidine, m.p. 142—144°C);
2-[(1′,3′-dimethylxanthin-7′-yl)methyl]-3-cyclopropylcarbonyl-thiazolidine, m.p. 210—212°C;
2-[(1′,3′-dimethylxanthin-7′-yl)methyl]-3-(2′-bromo)propionyl-thiazolidine, m.p. 220—222°C;
2-[(1′,3′-dimethylxanthin-7′-yl)methyl]-3-(3′-cycloexyl)propionyl-thiazolidine, m.p. 165—166°C;
2-[(1′,3′-dimethylxanthin-7′-yl)methyl]-3-(3′-cyclopentyl)propionyl-thiazolidine, m.p. 168—170°C;
2-[(1′,3′-dimethylxanthin-7′-yl)methyl]-3-ethyloxalyl-thiazolidine, m.p. 150—152°C.

Example 8

Under nitrogen atmosphere a solution of ethyl chlorocarbonate (1.14 ml) in anhydrous sym-dichloroethane (5 ml) is added dropwise to a stirred solution of acetylthioacetic acid (1.6 g) and triethylamine (1.7 ml) in anhydrous sym-dichloroethane cooled at −20°C.

After one hour the mixture is treated with a solution of 2-[(1′,3′-dimethylxanthin-7′-yl)methyl]-thiazolidine (2.8 g) in anhydrous sym-dichloroethane (10 ml) and then heated to room temperature. After three hours, water and ice are added. The organic phase is washed with 5% $NaHCO_3$ and water until neutral, dried on sodium sulphate and evaporated under vacuum. By crystallization from ethanol 2.4 g of 2-[(1′,3′-dimethylxanthin-7′-yl)methyl]-3-acetylthioacetyl-thiazolidine, m.p. 202—204°C, are obtained.

Example 9

2.4 Grams of potassium thioacetate are added to a stirred suspension (3.5 g) of 2-[(3′,7′-dimethylxanthin-1′-yl)methyl]-3-chloroacetyl-thiazolidine in anhydrous acetone (100 ml). After 3 hours the solvent is evaporated and the residue is partitioned between water (100 ml) and chloroform (150 ml). The organic phase is washed with 5% aqueous $NaHCO_3$ and water until netural, and then evaporated to dryness. The residue is crystallized from ethanol to give 3.2 g of 2-[(3′,7′-dimethylxanthin-1′-yl)methyl]-3-acetylthioacetyl-thiazolidine, m.p. 116—120°C.

Example 10

Under nitrogen atmosphere a solution of 0.9 g of 2-[(2′,4′-dimethylxanthin-7′-yl)methyl]-3-α-chloroacetyl-thiazolidine in 50 ml of dichloroethane is treated with 0.55 g of thiobenzoic acid, 0.15 g of tetrabutylammonium bromide and a solution of 0.53 g of potassium carbonate in 25 ml of water. The mixture is stirred overnight. The organic phase is washed with 2 × 25 ml of water, dried on sodium sulphate, and the solvent is evaporated in vacuum, to give 0.85 g of 2-[(1′,3′-dimethylxanthin-7′-yl)methyl]-3-benzoylthioacetyl-thiazolidine, m.p. 210—213°C (from ethanol).

Example 11

Using in the procedure of the Example 10 the benzoyl, the cyclohexylpropionyl, the cyclopentylpropionyl and the 3,4-diacetoxy-cinnamoyl esters of the α-mercaptoacetic acid and by reaction with the 2-[(1′,3′-dimethylxanthin-7′-yl)methyl]thiazolidine and 2-[(3′,7′-dimethylxanthin-1′-yl)methyl]thiazolidine, the following compounds are prepared:

2-[(3′,7′-dimethylxanthin-1′-yl)methyl]-3-benzoyl-thioacetyl-thiazolidine, m.p. 220—223°C;
2-[(3′,7′-dimethylxanthin-1′-yl)methyl]-3-(3′-cyclopentylpropionyl-thio)acetyl-thiazolidine, m.p. 210—213°C;
2-[(3′,7′-dimethylxanthin-1′-yl)methyl]-3-(3′-cyclopentylpropionyl-thio)acetyl-thiazolidine, m.p. 215—217°C;
2-[(1′,3′-dimethylxanthin-7′-yl)methyl]-3-(3′-cycloexylpropionyl-thio)acetyl-thiazolidine, m.p. 200—203°C;
2-[(1′,3′-dimethylxanthin-7′-yl)methyl]-3-(3,4-diacetoxy-cinnamoyl-thio)acetyl-thiazolidine, m.p. 200—205°C.

Example 12

A stirred suspension of 2-[(1',3'-dimethylxanthin-7'-yl)methyl]-3-α-chloroacetyl-thiazolidine (3.5 g) and sodium iodide (2.1 g) in 50 ml of methylethylketone is refluxed for 12 hours. After removal of the solvent the residue is partitioned between chloroform and water. The organic phase is washed with aqueous sodium bicarbonate and sodium thiosulphate, and once more with water. The residue obtained after evaporation of the solvent is crystallized from ethanol, to give 3.4 g of 2-[(1',3'-dimethylxanthin-7'-yl)-methyl]-3-α-iodoacetyl-thiazolidine, m.p. 210—212°C.

Example 13

In inert gas atmosphere, aqueous 30% ammonium hydroxyle (1.5 ml) is added to a stirred solution of 2-[(1',3'-dimethylxanthin-7'-yl)methyl]-3-acetylthioacetyl-thiazolidine (0.5 g) in dimethoxyethane. After 2 hours the mixture is diluted with water, the ammonia excess is removed under vacuum and the solid obtained is filtered. After washing with ethanol 0.25 g of 2-[(1',3'-dimethylxanthin-7'-yl)methyl]-3-α-mercapto-acetyl-thiazolidine, m.p. 276—278°C are obtained.

In a similar way the 2-[(3',7'-dimethylxanthin-1'-yl)methyl]-3-α-mercaptoacetyl-thiazolidine, m.p. 271—273°C, is prepared.

Example 14

A solution of isobutylchlorocarbonate (1.44 ml) in methylene chloride (6 ml) is dropwise added to a solution of (4-methyl-piperazine-1-yl)acetic acid (1.73 g) and triethylamine (1.52 ml) in methylene chloride (50 ml), cooled at −20°C. The mixture is kept at the temperature of −20°C for 45 minutes, then a solution of 2-[(1',3'-dimethylxanthin-7'-yl)methyl]-3-mercaptoacetyl-thiazolidine (4.6 g) in methylene chloride is added. The mixture is kept at −20°C for 45 minutes and then it is heated at room temperature. After the usual work-up the 2-[(1',3'-dimethylxanthin-7'-yl)methyl]-3-(4'-methylpiperazin-1'-yl)acetyl-thioacetyl)-thiazolidine, m.p. 212—214°C, is obtained.

In similar way the 2-[(3',7'-dimethylxanthin-1'-yl)methyl]-3-(4'-methylpiperazin-1'-yl)acetyl-thioacetyl-thiazolidine, m.p. 208—210°C, is obtained.

Example 15

A solution of 2-[(1',3'-dimethylxanthin-7'-yl)methyl]-3-chloroacetyl)thiazolidine (1 g) in DMSO (10 ml) is treated with 0.5 g of sodium imidazole. After overnight stirring at room temperature, the mixture is diluted with water and extracted with chloroform. After the usual work-up and crystallization from ethylether, 0.6 g of 2-[(1',3'-dimethylxanthin-7'-yl)methyl]-3-(imidazol-1-yl)acetyl-thiazolidine, m.p. 235—238°C, are obtained.

With the same procedure, the 2-[(3',7'-dimethylxanthin-1'-yl)methyl]-3-(imidazole-1'-yl)acetyl-thiazolidine, m.p. 231—234°C, is prepared.

Example 16

Succinic anhydride (0.48 g) is added to a solution of 2-[(3',7'-dimethylxanthin-1'-yl)methyl]thiazolidine (1.9 g) and dimethylaminopiridine (63 mg) in anhydrous piridine. After a night the solution is diluted with water and acidified with $H_2SO_4$2N. The aqueous phase is extracted with chlorofrom and the collected organic phases are washed with water. After evaporation of the solvent, the residue is crystallized from methanol/ethyl ether, to give 0.4 g of 2-[(3',7'-dimethylxanthin-1'-yl)methyl]-3-(3'-carboxypropionyl)-thiazolidine, m.p. 200—202°C.

In a similar way, the 2-[(1',3'-dimethylxanthin-7'-yl)methyl]-3-(3'-carboxypropionyl)thiazolidine, m.p. 208—210°C, is prepared.

Example 17

By using potassium thioacetate in the procedure of Example 15 instead of soidum imidazole, the 2-[(3',7'-dimethylxanthin-1'-yl)methyl]-3-acetylthioacetyl-thiazolidine, m.p. 116—120°C, is obtained.

Example 18

By using the potassium thiobenzoate in the procedure of Example 15, instead of sodium imidazole, the 2-[(1',3'-dimethylxanthin-7'-yl)methyl]-3-benzoylthioacetylthiazolidine, m.p. 210—213°C, is obtained.

Example 19

1.38 Grams of potassium carbonate and 1.275 ml of N-methylpiperazine are added to a solution of 2-[(1',3'-dimethylxanthin-7'-yl)methyl]-3-α-chloroacetyl-thiazolidine (3.57 g) in 50 ml of anhydrous acetonitrile. The suspension is refluxed for one hour under stirring. After removal of the solvent the residue is partitioned between water and chloroform, the organic phase is washed with water, dried on sodium sulphate and concentrated under vacuum. The residue is crystallized from ethyl ether to give 2.85 g of 2-[(1',3'-dimethylxanthin-7'-yl)methyl]-3-(4'-methylpiperazin-1'-yl)acetyl-thiazolidine, m.p. 222—225°C.

Example 20

By using in the procedure of Example 19 the 2-[(3',7'-dimethylxanthin-1'-yl)methyl]-3-α-chloroacetyl-thiazolidine as starting material, the 2-[(3',7'-dimethylxanthin-1'-yl)methyl]-3-α-(4'-methylpiperazin-1'-yl)-acetyl-thiazolidine, m.p. 175—180°C, is prepared.

Example 21

1.23 Grams of triphenylphosphine are added to a solution of 2-[(1′,3′-dimethylxanthin-7′-yl)methyl]-3-α-chloroacetyl-thiazolidine (1.3 g) in anhydrous acetonitrile (15 ml). The mixture is heated to reflux for 4 hours and diluted after cooling with 15 ml of ethyl ether. The crystalline precipitate is filtered and washed with ethyl ether, to give 2 g of 2-[(1′,3′-dimethylxanthin-7′-yl)methyl]-3-α-triphenylphosphonium-acetyl-thiazolidine chloride, m.p. 238—240°C. 30 Ml of methylene chloride and some drops of a phenolphatleine alcoholic solution are added to a solution of this product in 30 ml of water. Then an aqueous 0.1 N sodium hydroxyde is added, until a persistant pink colour is obtained. The organic phase is separated, washed with water and evaporated to dryness to give 1.6 g of 2-[(1′,3′-dimethylxanthin-7′-yl)methyl]-3-triphenyl-phospilidenemethylcarbonyl-thiazolidine, m.p. 122—126°C. A solution of 0.9 g of this compound in 6 ml of dimethylsulphoxide is treated with 0.3 g of 4-acetoxy-3-methoxybenzaldehyde. After two hours at room temperature the solution is diluted with 100 ml of water and extracted with methylene chloride. By elimination of the solvent, the residue is crystallized from ethyl acetate, to give 0.7 g of 2-[(1′,3′-dimethylxanthin-7′-yl)methyl]-3-(4′-acetoxy-3′-methoxy)cinnamoyl-thiazolidine, m.p. 208—210°C.

Example 22

Under inert gas atmosphere, ethyl chlorocarbonate (0.44 ml) in methylene chloride (2 ml) is added to a stirred solution of 3,4-diacetoxycinnamic acid (1 g) and triethylamine (0.55 ml) in methylene chloride (15 ml). The resulting mixture is stirred at −20°C for one hour. A solution of 2-[(1′,3′-dimethylxanthin-7′-yl)-methyl]thiazolidine (1..02 g) in 10 ml of methylene chloride is then added dropwise and the mixture stirred overnight at room temperature. After the usual work-up the residue is crystallized from ethyl ether, to give 0.9 g of 2-[(1′,3′-dimethylxanthin-7′-yl)methyl]-3-(3′,4′-diacetoxy)cinnamoylthiazolidine, m.p. 190—195°C

Example 23

Finely ground potassium carbonate (0.32 g) is added to a solution of 2-[(1′,3′-dimethylxanthin-7′-yl)-methyl]-3-(3′,4′-diacetoxy-cinnamoyl)thiazolidine (0.5 g) in 5 ml of ethanol and the suspension is stirred for 2 hours. After solvent evaporation under vacuum, the residue is partitioned between aqueous $NaH_2PO_4$ and chloroform. After washing with water to neutrality, the organic phase is dried on sodium sulphate and concentrated to dryness under vacuum. The obtained residue is crystallized from ethyl ether, to give 0.2 g of 2-[(1′,3′-dimethylxanthin-7′-yl)-methyl]-3-(3′,4′-dihydroxy)cinnamoyl-thiazolidine, m.p. 195—199°C.

Example 24

Using the same procedure described in Examples 22 and 23, from 2-[(3′,7′-dimethylxanthin-1′-yl)-methyl]thiazolidine, the 2-[(3′,7′-dimethylxanthin-1′-yl)methyl]-3-(3′,4′-dihydroxy)cinnamoyl-thiazolidine, m.p. 205—207°C, is obtained.

Example 25

A solution of dicyclohexylcarbodiimide (2.26 g) in DMF (10 ml) is added to a stirred suspension of 2-[(1′,3′-dimethylxanthin-7′-yl)methyl]thiazolidine (2.81 g), N-acetylcysteine (1.28 g) and 4-dimethylaminopiridine in DMF (25 ml), cooling at 0°C. After 12 hours, the dicyclohexylurea is filtered out and the solvent concentrated under vacuum. The residue after the usual work-up is crystallized with isopropanol to give 2-[(1′,3′-dimethylxanthin-7′-yl)methyl]-3-(N-acetylglycinyl)thiazolidine (3.5 g), m.p. 204—205°C.

Using the same procedure with 2-[(3′,7′-dimethylxanthin-1′-yl)methyl]thiazolidine, the 2-[(3′,7′-dimethylxanthin-1′-yl)methyl]-3-(N-acetylglycinyl)thiazolidine is obtained.

Example 26

Using in the procedure of Example 25, 25 g of N-formyl-glycine, by reaction with the appropriate thiazolidine, the following products are obtained:

2-[(1′,3′-dimethylxanthin-7′-yl)methyl]-3-(N-formylglycinyl)thiazolidine; and

2-[(3′,7′-dimethylxanthin-1′-yl)methyl]-3-(N-formylglycinyl)thiazolidine.

By treatment of a solution of the first N-formyl-glycinyl derivative in methanol with 8N HCl solution in isopropanol for 8 hours at room temperature, followed by concentration of the mixture at one third of the initial volume, the 2-[(1′,3′-dimethylxanthin-7′-yl)methyl]-3-(glycinyl)thiazolidine hydrochloride is obtained.

Example 27

A stirred solution of 3,5-dibromo-salicylaldehyde (2.79 g), 2-[(1′,3′-dimethylxanthin-7′-yl)methyl]-3-(glycinyl)thiazolidine (3.6 g) and triethylamine (1.38 ml) in methanol (250 ml) is heated at the reflux temperature for 3 hours and then cooled at room temperature. The stirring is continued for 8 hours in order to precipitate 2-[(1′,3′-dimethylxanthin-7′-yl)methyl]-3-[2′-(3″,5″-dibromo-2″-hydroxy-benzylidene-amino)acetyl/thiazolidine. 10% $NaBH_4$ supported on alumina (8 g) is added to a stirred solution of this compound (5.3 g) in dichloroethane (250 ml). After 6 hours the organic phase is filtered, washed with water and dried on $Na_2SO_4$.

Subsequent treatment with 8N HCl in isopropanol (4.8 ml) gives: 2-[(1′,3′-dimethylxanthin-7′-yl)-methyl]-3-[(3′,5′-dibromo-6′-hydroxyphenyl)methyl-aminoacetyl]thiazolidine HCl, m.p. 251—252°C (4.7 g).

Example 28

Acryloyl chloride (0.88 ml) is added dropwise to a stirred mixture of 2-[(1',3'-dimethylxanthin-7'-yl)-methyl]thiazolidine (2.8 g), triethylamine (1.52 ml) and anhydrous methylene chloride (50 ml), cooled at 0°C. The mixture is warmed at room temperature, then heated to reflux for 1 hour. The organic phase is washed with water, 5% sodium hydroxyde and then with water. After drying on $Na_2SO_4$, the solvent is evaporated to dryness under vacuum and the crude material is crystallized from isopropanol alcohol to give 2-[(1',3'-dimethylxanthin-7'-yl)methyl]-3-(propenoyl)thiazolidine (2.7 g), m.p. 190—192°C.

Example 29

2-(2-Aminoethylamino)ethanol (0.21 ml) is added to a solution of 2-[(1',3'-dimethylxanthin-7'-yl)-methyl]-3-(propenoyl)thiazolidine (0.67 g) in ethanol (10 ml) and the solution is refluxed for 2 hours. The solvent is then evaporated to dryness under vacuum and the crude material is dissolved with methylene chloride (20 ml); the solution is washed with water (2 × 10 ml), dried on $Na_2SO_4$ and the solvent is evaporated to dryness under vacuum.

The crude solid is crystallized from ethanol to give 2-[(1',3'-dimethylxanthin-7'-yl)methyl]-3-[3'-(2''-hydroxyethyl-aminoethyl-amino)propionyl]thiazolidine, m.p. 140—142°C.

Using in this procedure N-(2'-hydroxyethyl)piperazine and imidazole, the 2-[(1',3'-dimethylxanthin-7'-yl)methyl]-3-[3'-(2''-hydroxyethylpiperazin-1''-yl)propionyl]thiazolidine, and 2-[(1',3'-dimethylxanthin-7'-yl)methyl]-3-[3'-(imidazol-1''-yl)propionyl]thiazolidine, are prepared.

Example 30

N,N'-dicyclohexylcarbodiimide (2.26 g) is added to a stirred solution of 2-[(3',7'-dimethylxanthin-1'-yl)-methyl]thiazolidine (2.81 g), 3-(2'-morpholinoethyl)thioacetic acid (2.19 g) and 4-dimethylaminopiridine (0.12 g) in DMF (30 ml) cooled at 0°C. After 12 hours, the dicyclohexylurea is removed by filtration, and the organic phase concentrated under vacuum and the residue dissolved in chloroform (150 ml), is washed with 5% aqueous $NaHCO_3$, water and then dried on $Na_2SO_4$. After removal of the solvent in vacuum, the residual oil is crystallized from ethanol to give 2-[(1',3'-dimethylxanthin-7'-yl)methyl]-3-[3'-(2''-morpholinoethyl)thioacetyl]thiazolidine (3.5 g).

In a similar manner the 2-[(1',3'-dimethylxanthin-7'-yl)methyl]-3-[2'-(4''-methyl-piperazinoethyl)thioacetyl]thiazolidine is prepared.

Example 31

A stirred solution of 2-[(1',3'-dimethylxanthin-7'-yl)methyl]-3-(triphenylphosphilydene-methyl-carbonyl)thiazolidine (0.9 g) in anhydrous DMSO (6 ml) is treated with 3-morpholinylmethyl-4-hydroxy-5-methoxy-benzaldehyde (0.38 g) at room temperature. After 24 hours the mixture is diluted with chloroform (50 ml) and washed with water. The organic phase is dried on $Na_2SO_4$ and concentrated under vacuum. After several crystallizations from ethyl acetate and then from ethanol, pure 2-[(1',3'-dimethylxanthin-7'-yl)methyl]-3-[(3-morpholinylmethyl-4'-hydroxy-5'-methoxy)cinnamoyl]thiazolidine (0.7 g) m.p. 182—184°C is obtained.

In similar way the following compounds are prepared:

2-[(1',3'-dimethylxanthin-7'-yl)methyl]-3-[(3'-pyrrolidinylmethyl-4'-hydroxy-5'-methoxy)cinnamoyl]-thiazolidine;

2-[(1',3'-dimethylxanthin-7'-yl)methyl]-3-[(3'-dimethylxamino-methyl-4'-hydroxy-5'-methoxy)-cinnamoyl]thiazolidine.

Example 32

Using in the procedure of the Example 31 the aldehydes:

2-(4'-methylpiperazin-1'-yl)ethanal, 2-(morpholin-1'-yl)-ethanal and p-fluorobenzaldehyde, the following compounds are obtained:

2-[(1',3'-dimethylxanthin-7'-yl)methyl]-3-(5-morpholinyl)-2-butenoyl-thiazolidine;

2-[(1',3'-dimethylxanthin-7'-yl)methyl]-3-[(4'-methylpiperazin-1'-yl)-2-butenoyl]thiazolidine;

2-[(1',3'-dimethylxanthin-7'-yl)methyl]-3-(4'-fluoro)-cinamoyl-thiazolidine.

Example 33

Using in the procedures of Examples 25 and 26 the following acids: phenoxy acetic, (2'-ethoxy-ethyloxy)acetic and (2'-diethylamino-ethyloxy)acetic, the following products are prepared:

2-[(1',3'-dimethylxanthin-7'-yl)methyl]-3-phenoxyacetyl-thiazolidine;

2-[(1',3'-dimethylxanthin-7'-yl)methyl]-3-[(2'-ethoxy)ethyloxyacetyl]thiazolidine;

2-[(1',3'-dimethylxanthin-7'-yl)methyl]-3-[2-diethylamino)ethyloxy-acetyl]thiazolidine.

Example 34

In inert gas atmosphere, cysteamine hydrochloride (1.13 g) is treated with 2-[(1',3'-dimethylxanthin-7'-yl)methyl]-3-propenoyl-thiazolidine (2.6 g) in methanol (100 ml) for 8 hours at the reflux temperature.

The reaction mixture is cooled and after two days the crystalline precipitate is filtered to give 3.5 g of 2-[(1',3'-dimethylxanthin-7'-yl)methyl]-3-[3'-(2''-amino-ethylthio)propionyl]thiazolidine hydrochloride.

Using in the same procedure N-acetyl-cysteine, in the presence of catalytic amount of sodium methylate, the 2-[(1',3'-dimethylxanthin-7'-yl)methyl]-3-[(4'-thia-5'-carboxy-5-acetylamino)hexanoyl]-thiazolidine, m.p. 240—242°C, is prepared.

Example 35

A solution of N-acetylcysteine disodium salt (2.5 g) in MeOH (20 ml) is treated with a solution of 2-[(1',3'-dimethylxanthin-7'-yl)methyl]-3-chloroacetyl-thiazolidine (3 g) in DMSO (10 ml). After 3 hours at room temperature, the mixture is evaporated to small volume and the residue is partitioned between chloroform and aqueous 20% $NaH_2PO_4$ solution. The organic phase after the usual work-up gives 2 g of 2-[(1',3'-dimethylxanthin-7'-yl)methyl]-3-(3'-thia-5'-carboxy-5'-acetylamino-pentanoyl)thiazolidine.

**Claims for the Contracting States: BE CH DE FR GB IT LI LU NL SE**

1. Compounds of formula I

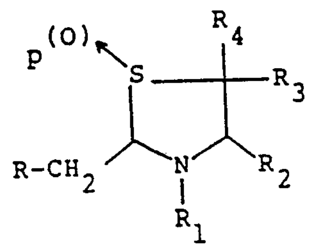

(I)

wherein:

R is 1,3-dimethylxanthin-7-yl or 3,7-dimethylxanthin-1-yl;

$R_2$ is hydrogen, a carboxy group or, when p=o and $R_1=R_3=R_4=H$, a carboxy group esterified with $C_1$—$C_5$-alcohols,

both $R_3$ and $R_4$ are hydrogen or methyl;

p is zero or 1;

$R_1$ is: hydrogen, a $C_1$—$C_2$ alkyl sulphonyl group, an unsubstituted or mono or poly-substituted phenyl sulphonyl group, or an acyl group of formula

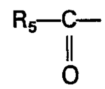

wherein $R_5$ is: hydrogen,

$-(CH_2)_n-Q$ and 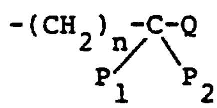

wherein n is 0 or an integer from 1 to 7; $P_1$, $P_2$, are both hydrogen or one of them is hydrogen and the other one is lower $C_1$—$C_4$ alkyl or phenyl and Q is selected in the group consisting of: hydrogen; a $C_3$—$C_4$-branched alkyl; a $C_3$—$C_7$ cycloalkyl; free carboxy group or $COOC_2H_5$ when $R_2=R_3=R_4=H$; an halogen atom; SH; —$NH_2$; a mono or di-substituted amino, t-butoxy carbonylamino or $C_1$—$C_2$ acylamino group; an ether —O—T or thioether S—T chain, wherein T is an unsubstituted or mono- or polysubstituted phenyl ring or a group of formula $(CH_2)_m$—$T_1$, wherein $T_1$ is selected in the group consisting of H, OH, $OCH_3$, $OC_2H_5$, $HOCH_2$—$CH_2$—, free carboxy group or $COOC_2H_5$ when $R_2=R_3=R_4=H$; $NH_2$, a $C_1$—$C_2$-acylamino or mono- or disubstituted amino group, or a group of formula

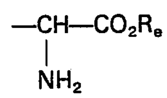

wherein $R_e$ is hydrogen, methyl or ethyl, and m is an integer from 1 to 3; a phenyl, phenoxy or phenylthio ring unsubstituted or mono- or polysubstituted in the m, o, and p-positions;

a group of formula —$(CH_2)_m$—SCO—$(CH_2)_nP_3$ wherein m and n have the above defined meanings and $P_3$ is a lower $C_1$—$C_7$, linear or branched alkyl chain, a $C_3$—$C_6$-cycloalkyl, a disubstituted amino group, a phenyl or phenoxy ring, optionally mono- or polysubstituted in the o, m and p-positions; an alkenyl chain of formula

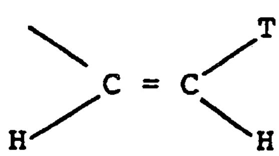

wherein T, in addition to the above defined meanings, may also be hydrogen;

the term "mono substituted amino group" comprises, within the meanings thereof, an amino group substituted by a $C_1$—$C_6$, linear or branched alkyl group or by groups having formula:

$$-CH_2-CH_2-O-CH_2-CH_3, \quad -CH_2-CH_2-O-CH_2-CH_2-OH,$$

$$-CH_2-CH_2-NH-CH_2-CH_3, \quad -CH_2-CH_2-NH-CH_2-CH_2-OH \text{ or}$$

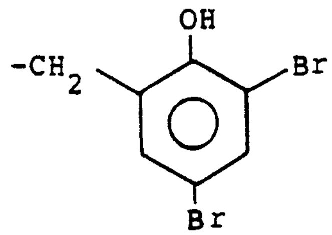

the substituents of a disubstituted amino group according to the invention may be linear or branched $C_1$—$C_6$ alkyl groups or, taken together, they represent an unsaturated or saturated nitrogen ring such as morpholin-1-yl, pyrrolidin-1-yl, piperidin-1-yl, 4-methyl-piperazin-1-yl, thiomorpholin-1-yl, 4-ethyl-piperazin-1-yl, 4-(2'-fluorophenyl)piperazin-1-yl; imidazol-1-yl, 3-pyridyl, 4-pyridyl;

finally, the term "mono- or polysubstituted phenyl", according to the invention, means phenyl groups which are substituted by a fluorine atom in the para position, by chlorine atoms in the meta and/or para positions or by a $CF_3$ in the meta positions, or a phenyl group of formula

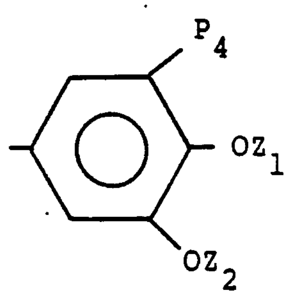

wherein $Z_1$ is H or $COCH_3$ and $Z_2$ is H, $CH_3$ or $COCH_3$ and $P_4$ is selected in the group consisting of hydrogen, aminomethyl, $C_1$—$C_2$-acylaminomethyl or mono- or di-substituted aminomethyl group, as above defined.

2. Compounds of formula I wherein $R_2$, $R_3$ and $R_4$ are hydrogen and $R_1$ is an acyl group of formula $R_5CO$ wherein $R_5$ has the above defined meanings.

3. Compounds of formula I wherein $R_2$, $R_3$ and $R_4$ are hydrogen and $R_1$ is a substituted or unsubstituted cinnamoyl group.

4. A compound selected in the group consisting of:

2-[(1',3'-dimethylxanthin-7'-yl)methyl]thiazolidin-4-carboxylic acid and their methyl and tert-butyl esters;

2-[(3',7'-dimethylxanthin-1'-yl)methyl]thiazolidin-4-carboxylic acid and their methyl, ethyl and tert-butyl esters;

2-[(1',3'-dimethylxanthin-7'-yl)methyl]thiazolidine;

2-[(3',7'-dimethylxanthin-1'-yl)methyl]thiazolidine;

2-[(1',3'-dimethylxanthin-7'-yl)methyl]-3-(imidazol-1'-yl)acetyl]thiazolidine;

2-[(3',7'-dimethylxanthin-1'-yl)methyl]-3-[(imidazol-1'-yl)acetyl]thiazolidine;

2-[(1',3'-dimethylxanthin-7'-yl)methyl]-3-[(3',4'-hydroxy)cinnamoyl]thiazolidine;

2-[(3',7'-dimethylxanthin-1'-yl)methyl]-3-[(3',4'-dihydroxy)cinnamoyl]thiazolidine;

2-[(1',3'-dimethylxanthin-7'-yl)methyl]-3-(3'-methoxy-4'-hydroxy)cinnamoyl-thiazolidine;

2-[(3',7'-dimethylxanthin-1'-yl)methyl]-3-(3'-methoxy-4'-hydroxy)cinnamoyl-thiazolidine;

2-[(3',7'-dimethylxanthin-1'-yl)methyl]-3-[3'-(4''-hydroxyethylpiperazin-1''-yl)propionyl]thiazolidine;

2-[(1',3'-dimethylxanthin-7'-yl)methyl]-3-(3'-morpholinomethyl-4'-hydroxy-3'-methoxy-cinnamoyl)thiazolidine;

2-[(1',3'-dimethylxanthin-7'-yl)methyl]-3-(3'-pyrrolidyl-methyl-4'-hydroxy-3'-methoxy-cinnamoyl)thiazolidine;

2-[(3',7'-dimethylxanthin-1'-yl)methyl]-3-(3'-pyrrolidinylmethyl-4'-hydroxy-3'-methoxy-cinnamoyl)thiazolidine;

2-[(1',3'-dimethylxanthin-7'-yl)methyl]-3-[3'-thia-5'-carboxy-5'-acetylamino-pentanoyl]thiazolidine;

2-[(1',3'-dimethylxanthin-7'-yl)methyl]-3-[4'-thia-6'-carboxy-6'-acetyl-amino-hexanoyl]thiazolidine;

2-[(1',3'-dimethylxanthin-7'-yl)methyl]-3-[3'-(2''-ethoxyethyloxy)acetyl]thiazolidine;
2-[(1',3'-dimethylxanthin-7'-yl)methyl]-3-[3'-(imidazol-1'-yl)propionyl]thiazolidine;
2-[(1',3'-dimethylxanthin-7'-yl)methyl]-3-glycinyl-thiazolidine;
2-[(3',7'-dimethylxanthin-1'-yl)methyl]-3-glycinyl-thiazolidine;
2-[(1',3'-dimethylxanthin-7'-yl)methyl]-3-(N-acetyl)glycinyl-thiazolidine;
2-[(1',3'-dimethylxanthin-7'-yl)methyl]-3-[3'-(2''-hydroxyethylamino)ethylamino-propionyl]thiazolidine;
2-[(3',7'-dimethylxanthin-1'-yl)methyl]-3-[3'-(2''-hydroxyethylamino)ethylamino-propionyl]thiazolidine;
2-[(1',3'-dimethylxanthin-7'-yl)methyl]-3-[3'-(4''-hydroxyethylpiperazin-1''-yl)propionyl]thiazolidine.

5. A process for the preparation of compounds of formula I characterized in that a compound of formula II:

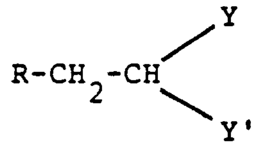

(II)

wherein R is as above defined and both Y and Y' are lower $C_1$—$C_2$ alkoxy or, taken together, form a carboxyl group, is reacted with an amino alkanethiol of formula III

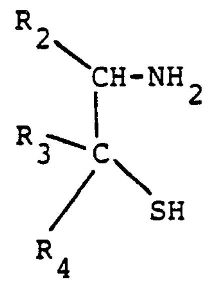

(III)

wherein $R_2$, $R_3$ and $R_4$ are as above defined, and that the obtained compounds of formula (Ia)

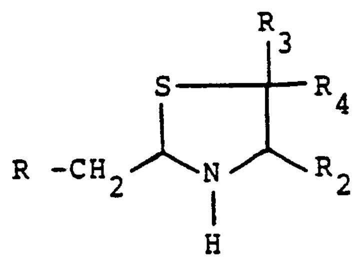

(Ia)

wherein R, $R_2$, $R_3$, $R_4$ are as above defined, are then optionally reacted with succinic or glutaric anhydride or a compound of formula

$Q-(CH_2)_n-COZ$ (IVa)

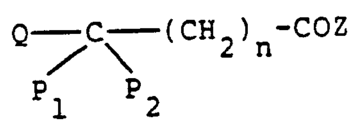

(IVb)

$P_5-SO_2-Hal$ (IVc)

wherein:

Q, $P_1$, $P_2$ and n are as above defined;

$P_5$ is a $C_1$—$C_2$-alkyl group or an unsubstituted or mono or polysubstituted phenyl ring;

Hal is an halogen atom and Z is an activating group of the carboxy moiety such as halogen, azide, activated esters or anhydrides.

6. A process for the preparation of compounds of formula I wherein $R_1$ is a group of formula

—CH=CH—T or 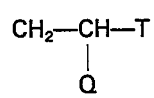

wherein T and Q are as above defined,
characterized in that a compound of formula Ie

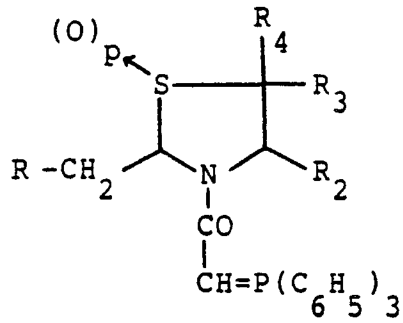

(Ie)

wherein R, $R_3$, $R_4$, $R_2$ and p are as above defined, are reacted with an aldehyde of formula V

T—CHO (V)

wherein T is as above defined and that the so obtained compounds are optionally subjected to Michael reaction with nucleophilic compounds selected in the group of amines of formula $H_2N—(CH_2)_m—T_1$; mono- or di-substituted amines or thiols of formula $HS—(CH_2)_n—T_1$, thiophenols optionally mono- or di-substituted, m, n and $T_1$ being as above defined.

7. Process for the preparation of compounds of formula I, wherein $R_1$ is a group of formula

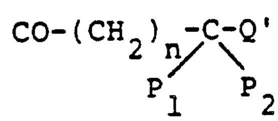

wherein n, $P_1$ and $P_2$ are as above defined, and Q′ is a SH, an alkylthioether group or a group of formula $P_3—(CH_2)_n—COS—$,
characterized in that a compound of formula Id

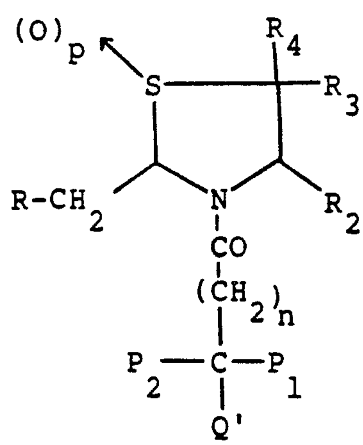

(Id)

wherein R, $R_2$, $R_3$, $R_4$, p are as above defined, at least one of $P_1$ and $P_2$ is hydrogen, and the other one is hydrogen, methyl or phenyl, Q′ is a halogen atom and n is preferably zero,
is reacted with a thiocarboxylic acid salt of the formula

$P_3—(CH_2)_n—COS^{(-)}$ Base$^{(+)}$

wherein $P_3$ and n are as above defined and the base is an alkaline or alkaline-earth metal, lower trialkylammonium or phenyldialkylammonium, and that the so obtained compounds are reacted with ammonia to give Q=SH which can be optionally alkylated with an alkyl halide in the presence of a base.

8. Pharmaceutical compositions having mucus regulating, bronchodilatory, antitussive activity containing a therapeutically effective amount of a compound as claimed in any of the claims 1—4 in admixture with non-toxic carriers or excipients.

**Claims for the Contracting State: AT**

1. A process for the preparation of compounds of formula I

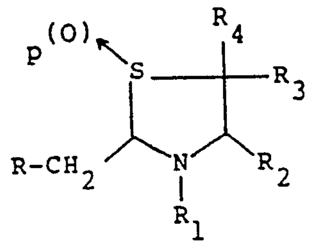

(I)

wherein:

R is 1,3-dimethylxanthin-7-yl or 3,7-dimethylxanthin-1-yl;

$R_2$ is hydrogen, a carboxy group or, when p=0 and $R_1=R_3=R_4=H$, a carboxy group esterified with $C_1$—$C_5$-alcohols,

both $R_3$ and $R_4$ are hydrogen or methyl;

p is zero or 1;

$R_1$ is: hydrogen, a $C_1$—$C_2$ alkyl sulphonyl group, an unsubstituted or mono or poly-substituted phenyl sulphonyl group, or an acyl group of formula

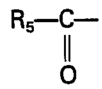

wherein $R_5$ is: hydrogen,

$-(CH_2)_n-Q$ and 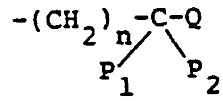

wherein n is 0 or an integer from 1 to 7; $P_1$, $P_2$, are both hydrogen or one of them is hydrogen and the other one is lower $C_1$—$C_4$ alkyl or phenyl and Q is selected in the group consisting of: hydrogen; a $C_3$—$C_4$-branched alkyl; a $C_3$—$C_7$ cycloalkyl; free carboxy group or $COOC_2H_5$ when $R_2=R_3=R_4=H$; an halogen atom; SH; —$NH_2$; a mono or di-substituted amino, t-butoxy carbonylamino or $C_1$—$C_2$ acylamino group; an ether —O—T or thioether S—T chain, wherein T is an unsubstituted or mono- or polysubstituted phenyl ring or a group of formula $(CH_2)_m$—$T_1$, wherein $T_1$ is selected in the group consisting of H, OH, $OCH_3$, $OC_2H_5$, $HOCH_2$—$CH_2$—, free carboxy group or $COOC_2H_5$ when $R_2=R_3=R_4=H$; $NH_2$, a $C_1$—$C_2$-acylamino or mono- or disubstituted amino group, or a group of formula

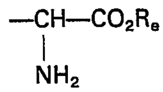

wherein $R_e$ is hydrogen, methyl or ethyl, and m is an integer from 1 to 3; a phenyl, phenoxy or phenylthio ring unsubstituted or mono- or polysubstituted in the m, o, and p-positions;

a group of formula —$(CH_2)_m$—SCO—$(CH_2)_nP_3$ wherein m and n have the above defined meanings and $P_3$ is a lower $C_1$—$C_7$, linear or branched alkyl chain, a $C_3$—$C_6$-cycloalkyl, a disubstituted amino group, a phenyl or phenoxy ring, optionally mono- or polysubstituted in the o, m and p-positions; an alkenyl chain of formula

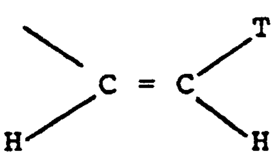

wherein T, in addition to the above defined meanings, may also be hydrogen;

the term "mono substituted amino group" comprises, within the meanings thereof, an amino group substituted by a $C_1$—$C_6$, linear or branched alkyl group or by groups having formula:

$-CH_2-CH_2-O-CH_2-CH_3$, $-CH_2-CH_2-O-CH_2-CH_2-OH$,

$-CH_2-CH_2-NH-CH_2-CH_3$, $-CH_2-CH_2-NH-CH_2-CH_2-OH$ or

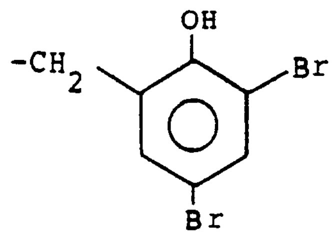

the substituents of a disubstituted amino group according to the invention may be linear or branched $C_1$—$C_6$ alkyl groups or, taken together, they represent an unsaturated or saturated nitrogen ring such as morpholin-1-yl, pyrrolidin-1-yl, piperidin-1-yl, 4-methyl-piperazin-1-yl, thiomorpholin-1-yl, 4-ethyl-piperazin-1-yl, 4-(2′-fluorophenyl)piperazin-1-yl; imidazol-1-yl, 3-pyridyl, 4-pyridyl;

finally, the term "mono- or polysubstituted phenyl", according to the invention, means phenyl groups which are substituted by a fluorine atom in the para position, by chlorine atoms in the meta and/or para positions or by a $CF_3$ in the meta positions, or a phenyl group of formula

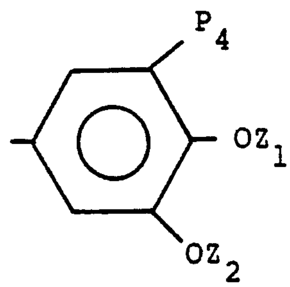

wherein $Z_1$ is H or $COCH_3$ and $Z_2$ is H, $CH_3$ or $COCH_3$ and $P_4$ is selected in the group consisting of hydrogen, aminomethyl, $C_1$—$C_2$-acylaminomethyl or mono- or di-substituted aminomethyl group, as above defined, characterized in that a compound of formula II

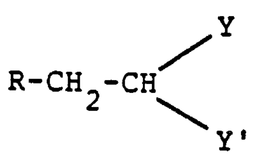

(II)

wherein R is as above defined and both Y and Y′ are lower $C_1$—$C_2$ alkoxy or, taken together, form a carbonyl group, is reacted with an amino alkanethiol of formula III

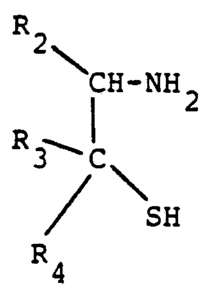

(III)

wherein $R_2$, $R_3$ and $R_4$ are as above defined, and that the obtained compounds of formula (Ia)

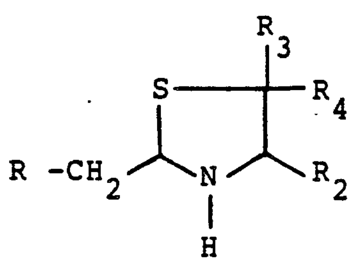

(Ia)

wherein R, $R_2$, $R_3$, $R_4$ are as above defined, are then optionally reacted with succinic or glutaric anhydride or a compound of formula

$Q-(CH_2)_n-COZ$ (IVa)

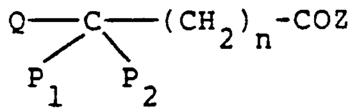

(IVb)

$P_5-SO_2-Hal$ (IVc)

wherein:

Q, $P_1$, $P_2$ and n are as above defined;

$P_5$ is a $C_1$—$C_2$-alkyl group or an unsubstituted or mono or polysubstituted phenyl ring;

Hal is an halogen atom and Z is an activating group of the carboxy moiety such as halogen, azide, activated esters or anhydrides.

2. A process for the preparation of compounds of formula I wherein $R_1$ is a group of formula

$$—CH{=}CH—T \text{ or } CH_2—CH(Q)—T$$

wherein T and Q are as above defined,
characterized in that a compound of formula Ie

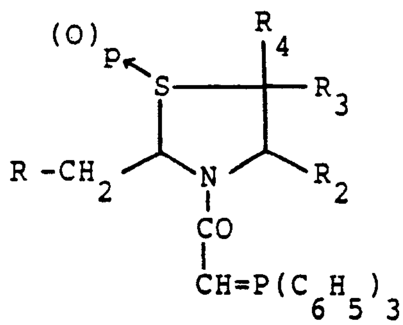

(Ie)

wherein R, $R_3$, $R_4$, $R_2$ and p are as above defined, are reacted with an aldehyde of formula V

T—CHO (V)

wherein T is as above defined and that the so obtained compounds are optionally subjected to Michael reaction with nucleophilic compounds selected in the group of amines of formula $H_2N—(CH_2)_m—T_1$; mono- or di-substituted amines or thiols of formula $HS—(CH_2)_n—T_1$, thiophenols optionally mono- or di-substituted, m, n and $T_1$ being as above defined.

3. Process for the preparation of compounds of formula I, wherein $R_1$ is a group of formula

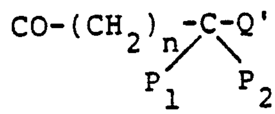

wherein n, $P_1$ and $P_2$ are as above defined, and Q' is a SH, an alkylthioether group or a group of formula $P_3—(CH_2)_n—COS—$,
characterized in that a compound of formula Id

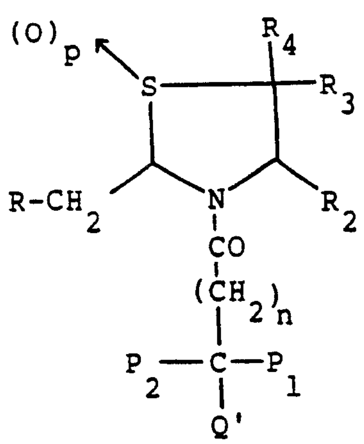

(Id)

wherein R, $R_2$, $R_3$, $R_4$, p are as above defined, at least one of $P_1$ and $P_2$ is hydrogen, and the other one is hydrogen, methyl or phenyl, Q' is a halogen atom and n is preferably zero, is reacted with a thiocarboxylic acid salt of the formula

$$P_3—(CH_2)_n—COS^{(-)}\ \ Base^{(+)}$$

wherein $P_3$ and n are as above defined and the base is an alkaline or alkaline-earth metal, lower trialkylammonium or phenyldialkylammonium, and that the so obtained compounds are reacted with ammonia to give Q=SH which can be optionally alkylated with an alkyl halide in the presence of a base.

**Patentansprüche für die Vertragsstaaten: BE CH DE FR GB IT LI LU NL SE**

1. Verbindungen der Formel I

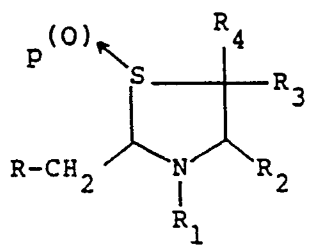

(I)

in der

R eine 1,3-Dimethylxanthin-7-yl- oder 3,7-Dimethylxanthin-1-ylgruppe bedeutet;

$R_2$ Wasserstoff, eine Carboxygruppe oder, wenn p=0 und $R_1=R_3=R_4=H$, eine mit $C_1—C_5$-Alkoholen veresterte Carboxygruppe darstellt;

sowohl $R_3$ als auch $R_4$ Wasserstoff oder eine Methylgruppe bedeuten;

p 0 oder 1 ist;

$R_1$ bedeutet: Wasserstoff, eine $C_1—C_2$-Alkylsulfonylgruppe, eine unsubstituierte oder mono- oder polysubstituierte Phenylsulfonylgruppe oder eine Acylgruppe der Formel

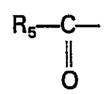

in der $R_5$ Wasserstoff,

$CH_2)_n$-Q und 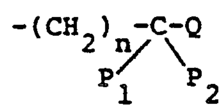

in der n 0 oder eine ganze Zahl von 1 bis 7 ist; $P_1$ und $P_2$ beide Wasserstoff sind oder eines von ihnen Wasserstoff und das andere $C_1—C_4$-Niederalkyl oder Phenyl bedeutet und Q in der Gruppe ausgewählt ist, die besteht aus Wasserstoff, ein verzweigter $C_3—C_4$-Alkylrest; ein $C_3—C_7$-Cycloalkylrest; freie Carboxylgruppe oder $COOC_2H_5$, wenn $R_2=R_3=R_4=H$; ein Halogenatom; SH; $—NH_2$; eine mono- oder disubstituierte Aminogruppe, eine tert.-Butoxycarbonylamino- oder $C_1—C_2$-Acylaminogruppe; eine Äther-O-T- oder Thioäther-S-T-Kette, in der T einen unsubstituierten oder mono- oder polysubstituierten Phenylring oder eine Gruppe der Formel $(CH_2)_m—T_1$ bedeutet, wobei $T_1$ in der Gruppe ausgewählt ist, die besteht aus H, OH, $OCH_3$, $OC_2H_5$, $HOCH_2—CH_2—$, freie Carboxylgruppe oder $COOC_2H_5$, wenn $R_2=R_3=R_4=H$, $NH_2$, eine $C_1—C_2$-Acylamino- oder mono- oder disubstituierte Aminogruppe, oder eine Gruppe der Formel

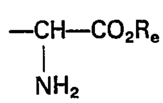

in der $R_e$ Wasserstoff, Methyl oder Äthyl ist und m eine ganze Zahl von 1 bis 3 ist; ein Phenyl-, Phenoxy- oder Phenylthioring, unsubstituiert oder mono- oder polysubstituiert in den m-, o- und p-Stellungen; eine Gruppe der Formel $—(CH_2)_m—SCO—(CH_2)_nP_3$ in der m und n die vorstehend angegebenen Bedeutungen haben und $P_3$ eine lineare oder verzweigte $C_1—C_7$-Niederalkylkette, ein $C_3—C_6$-Cycloalkylrest, eine disubstituierte Aminogruppe, ein Phenyl- oder Phenoxyring, gegebenenfalls mono- oder polysubstituiert in den o-, m- und p-Stellungen, eine Alkenylkette der Formel

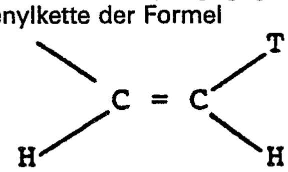

in der T zusätzlich zu den vorstehend angegebenen Bedeutungen auch Wasserstoff sein kann; der Begriff "monosubstituierte Aminogruppe" innerhalb seiner Bedeutungen eine durch einen linearen oder verzweigten $C_1$—$C_6$-Alkylrest oder durch Gruppen der Formel:

$$-CH_2-CH_2-O-CH_2-CH_3, \quad -CH_2-CH_2-O-CH_2-CH_2-OH,$$

$$-CH_2-CH_2-NH-CH_2-CH_3, \quad -CH_2-CH_2-NH-CH_2-CH_2-OH \text{ oder}$$

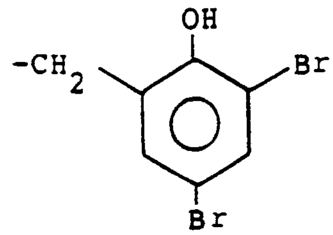

substituierte Aminogruppe umfaßt;
die Substituenten einer disubstituierten Aminogruppe gemäß der Erfindung lineare oder verzweigte $C_1$—$C_6$-Alkylreste sein können oder zusammengenommen einen ungesättigten oder gesättigten Stickstoff-Ring darstellen, wie Morpholin-1-yl, Pyrrolidin-1-yl, Piperidin-1-yl, 4-Methyl-piperazin-1-yl, Thiomorpholin-1-yl, 4-Äthylpiperazin-1-yl, 4-(2'-Fluorphenyl)-piperazin-1-yl, Imidazol-1-yl, 3-Pyridyl, 4-Pyridyl;

und schließlich der Begriff "mono- oder polysubstituierte Phenylgruppe" gemäß der Erfindung Phenylgruppen bedeutet, die durch ein Fluoratom in para-Stellung, durch Chloratome in den meta- und/oder para-Stellungen oder durch ein $CF_3$ in den meta-Stellungen substituiert sind, oder eine Phenylgruppe der Formel

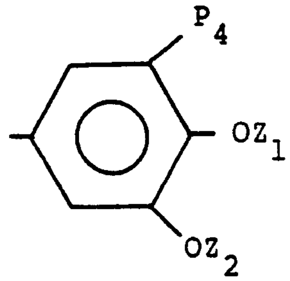

in der $Z_1$ H oder $COCH_3$ ist und $Z_2$ H, $CH_3$ oder $COCH_3$ bedeutet und $P_4$ in der Gruppe ausgewählt ist, die aus Wasserstoff, Aminomethyl, $C_1$—$C_2$-Acylaminomethyl oder mono- oder disubstituierte Aminomethylgruppen gemäß vorstehen der Definition besteht.

2. Verbindungen der Formel I, in denen $R_2$, $R_3$ und $R_4$ Wasserstoff sind und $R_1$ einen Acylrest der Formel $R_5CO$ bedeutet, in der $R_5$ die vorstehend angegebenen Bedeutungen hat.

3. Verbindungen der Formel I, in denen $R_2$, $R_3$ und $R_4$ Wasserstoff sind und $R_1$ eine substituierte oder unsubstituierte Cinnamoylgruppe ist.

4. Verbindung, ausgewählt in der Gruppe, die besteht aus:

2-[(1',3'-Dimethylxanthin-7'-yl)-methyl]-thiazolidin-4-carbonsäure und ihre Methyl- und tert.-Butylester;

2-[(3',7'-Dimethylxanthin-1'-yl)-methyl]-thiazolidin-4-carbonsäure und ihre Methyl-, Äthyl- und tert.-Butylester;

2-[(1',3'-Dimethylxanthin-7'-yl)-methyl]-thiazolidin;

2-[(3',7'-Dimethylxanthin-1'-yl)-methyl]-thiazolidin;

2-[(1',3'-Dimethylxanthin-7'-yl)methyl]-3-(imidazol-1'-yl)-acetyl]-thiazolidin;

2-[(3',7'-Dimethylxanthin-1'-yl)-methyl]-3-[(imidazol-1'-yl)-acetyl]-thiazolidin;

2-[(1',3'-Dimethylxanthin-7'-yl)-methyl]-3-[(3',4'-hydroxy)-cinnamoyl]-thiazolidin;

2-[(3',7'-Dimethylxanthin-1'-yl)-methyl]-3-[(3',4'-dihydroxy)-cinnamoyl]-thiazolidin;

2-[(1',3'-Dimethylxanthin-7'-yl)-methyl]-3-(3'-methoxy-4'-hydroxy)-cinnamoyl-thiazolidin;

2-[(3',7'-Dimethylxanthin-1'-yl)-methyl]-3-(3'-methoxy-4'-hydroxy)-cinnamoyl-thiazolidin;

2-[(3',7'-Dimethylxanthin-1'-yl)-methyl]-3-[3'-(4''-hydroxyäthylpiperazin-1''-yl)-propionyl]-thiazolidin;

2-[(1',3'-Dimethylxanthin-7'-yl)-methyl]-3-(3'-morpholinomethyl-4'-hydroxy-3'-methoxy-cinnamoyl)-thiazolidin;

2-[(1',3'-Dimethylxanthin-7'-yl)-methyl]-3-(3'-pyrrolidyl-methyl-4'-hydroxy-3'-methoxy-cinnamoyl)-thiazolidin;

2-[(3',7'-Dimethylxanthin-1'-yl)-methyl]-3-(3'-pyrrolidinylmethyl-4'-hydroxy-3'-methoxy-cinnamoyl)-thiazolidin;

2-[(1',3'-Dimethylxanthin-7'-yl)-methyl]-3-[3'-thia-5'-carboxy-5'-acetylamino-pentanoyl]-thiazolidin;
2-[(1',3'-Dimethylxanthin-7'-yl)-methyl]-3-[4'-thia-6'-carboxy-6'-acetyl-amino-hexanoyl]-thiazolidin;
2-[(1',3'-Dimethylxanthin-7'-yl)-methyl]-3-[3'-(2''-äthoxyäthyloxy)-acetyl]-thiazolidin;
2-[(1',3'-Dimethylxanthin-7'-yl)-methyl]-3-[3'-(imidazol-1'-yl)-propionyl]-thiazolidin;
2-[(1',3'-Dimethylxanthin-7'-yl)-methyl]-3-glycinyl-thiazolidin;
2-[(3',7'-Dimethylxanthin-1'-yl)-methyl]-3-glycinyl-thiazolidin;
2-[(1',3'-Dimethylxanthin-7'-yl)-methyl]-3-(N-acetyl)-glycinyl-thiazolidin;
2-[(1',3'-Dimethylxanthin-7'-yl)methyl]-3-[3'-(2''-hydroxyäthylamino)-äthylamino-propionyl]-thiazolidin;
2-[(3',7'-Dimethylxanthin-1'-yl)-methyl]-3-[3'-(2''-hydroxyäthylamino)-äthylamino-propionyl]-thiazolidin;
2-[(1',3'-Dimethylxanthin-7'-yl)-methyl]-3-[3'-(4''-hydroxyäthylpiperazin-1''-yl)-propionyl]-thiazolidin.

5. Verfahren zur Herstellung der Verbindungen der Formel I, dadurch gekennzeichnet, daß eine Verbindung der Formel II

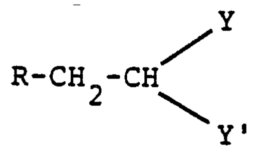

(II)

in der R wie vorstehend definiert ist und sowohl Y als auch Y' $C_1$—$C_2$-Niederalkoxyreste sind oder zusammengenommen eine Carbonylgruppe bilden, mit einem Aminoalkanthiol der Formel III

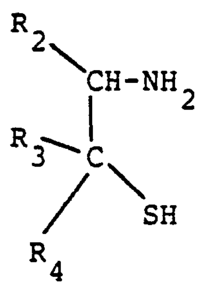

(III)

in der $R_2$, $R_3$ und $R_4$ wie vorstehend definiert sind, umgesetzt wird, und das die erhaltenen Verbindungen der Formel (Ia)

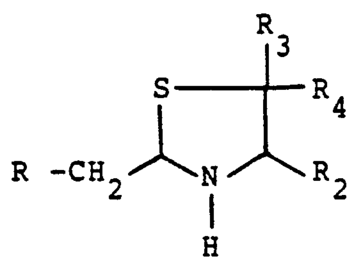

(Ia)

in der R, $R_2$, $R_3$ und $R_4$ wie vorstehend definiert sind, dann gegebenenfalls mit Bernsteinsäure- oder Glutarsäureanhydrid oder mit einer Verbindung der Formel umgesetzt werden

$Q-(CH_2)_n-COZ$ (IVa)

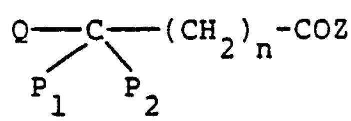

(IVb)

$P_5-SO_2-Hal$ (IVc)

wobei:
Q, $P_1$, $P_2$ und n wie vorstehend definiert sind;
$P_5$ einen $C_1$—$C_2$-Alkylrest oder einen unsubstituierten oder mono- oder polysubstituierten Phenylring bedeutet;
Hal ein Halogenatom ist und Z eine aktivierende Gruppe der Carboxyeinheit, wie Halogen, Azid, aktivierte Ester oder Anhydride, bedeutet.

6. Verfahren zur Herstellung von Verbindungen der Formel I, in denen $R_1$ eine Gruppe der Formel

—CH=CH—T oder 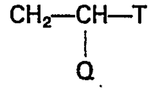

bedeutet, wobei T und Q wie vorstehend definiert sind, dadurch gekennzeichnet, daß eine Verbindung der Formel Ie

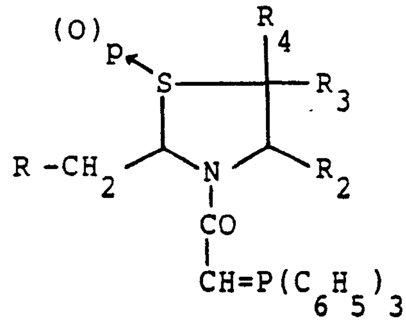

(Ie)

in der R, $R_3$, $R_4$, $R_2$ und p wie vorstehend definiert sind, mit einem Aldehyd der Formel V

T—CHO (V)

in der T wie vorstehend definiert ist, umgesetzt wird, und daß die so erhaltenen Verbindungen gegebenenfalls einer Michael-Reaktion mit nucleophilen Verbindungen unterzogen werden, die in der Gruppe der Amine der Formel $H_2N—(CH_2)_m—T_1$; mono- oder disubstituierten Amine oder Thiole der Formel $HS—(CH_2)_n—T_1$, oder gegebenenfalls mono- oder disubstituierten Thiophenole ausgewählt sind, wobei m, n und $T_1$ wie vorstehend definiert sind, unterzogen werden.

7. Verfahren zur Herstellung von Verbindungen der Formel I, in der $R_1$ eine Gruppe der Formel

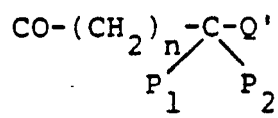

in der n, $P_1$ und $P_2$ wie vorstehend definiert sind und Q' eine SH—, eine Alkylthioäthergruppe oder eine Gruppe der Formel $P_3—(CH_2)_n—COS—$ bedeuten,
dadurch gekennzeichnet, daß eine Verbindung der Formel Id

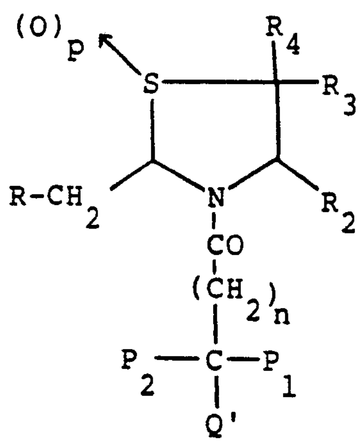

(Id)

in der R, $R_2$, $R_3$, $R_4$ und p wie vorstehend definiert sind, mindestens einer der Reste $P_1$ und $P_2$ Wasserstoff ist und der andere Wasserstoff, Methyl oder Phenyl bedeutet, Q' ein Halogenatom darstellt und n vorzugsweise 0 ist, mit einem Thiocarbonsäuresalz der Formel

$P_3—(CH_2)_n—\dot{C}OS^{(-)}$ $Base^{(+)}$

in der $P_3$ und n wie vorstehend definiert sind und die Base ein Alkali- oder Erdalkalimetall, eine niedere Trialkylammonium- oder Phenyldialkylammoniumgruppe bedeutet, umgesetzt wird, und daß die so erhaltenen Verbindungen mit Ammoniak umgesetzt werden, wobei Q=SH erhalten wird, das dann gegebenenfalls mit einem Alkylhalogenid in Gegenwart einer Base alkyliert werden kann.

8. Arzneimittel mit schleimregulierender, bronchodilatorischer und antitussiver Wirksamkeit, enthaltend eine therapeutisch wirksame Menge einer Verbindung nach einem der Ansprüche 1 bis 4 im Gemisch mit nicht toxischen Trägern oder Excipientien.

**Patentansprüche für den Vertragsstaat: AT**

1. Verfahren zur Herstellung von Verbindungen der Formel I

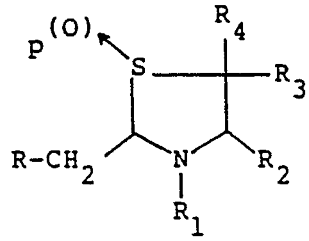

(I)

in der

R eine 1,3-Dimethylxanthin-7-yl- oder 3,7-Dimethylxanthin-1-ylgruppe bedeutet;

$R_2$ Wasserstoff, eine Carboxygruppe oder, wenn p=0 und $R_1=R_3=R_4=H$, eine mit $C_1$—$C_5$-Alkoholen veresterte Carboxygruppe darstellt;

sowohl $R_3$ als auch $R_4$ Wasserstoff oder eine Methylgruppe bedeuten;

p 0 oder 1 ist;

$R_1$ bedeutet: Wasserstoff, eine $C_1$—$C_2$-Alkylsulfonylgruppe, eine unsubstituierte oder mono- oder polysubstituierte Phenylsulfonylgruppe oder eine Acylgruppe der Formel

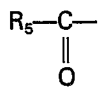

in der $R_5$ darstellt: Wasserstoff,

$-(CH_2)_n-Q$ und 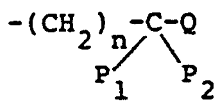

in der n 0 oder eine ganze Zahl von 1 bis 7 ist; $P_1$ und $P_2$ beide Wasserstoff sind oder eines von ihnen Wasserstoff und das andere $C_1$—$C_4$-Niederalkyl oder Phenyl bedeutet und Q in der Gruppe ausgewählt ist, die besteht aus Wasserstoff, ein verzweigter $C_3$—$C_4$-Alkylrest; ein $C_3$—$C_7$-Cycloalkylrest; freie Carboxylgruppe oder $COOC_2H_5$, wenn $R_2=R_3=R_4=H$; ein Halogenatom; SH; —$NH_2$; eine mono- oder disubstituierte Aminogruppe, eine tert.-Butoxycarbonylamino- oder $C_1$—$C_2$-Acylaminogruppe; eine Äther-O-T- oder Thioäther-S-T-Kette, in der T einen unsubstituierten oder mono- oder polysubstituierten Phenylring oder eine Gruppe der Formel $(CH_2)_m$—$T_1$ bedeutet, wobei $T_1$ in der Gruppe ausgewählt ist, die besteht aus H, OH, $OCH_3$, $OC_2H_5$, $HOCH_2$—$CH_2$—, freie Carboxylgruppe oder $COOC_2H_5$, wenn $R_2=R_3=R_4=H$, $NH_2$, eine $C_1$—$C_2$-Acylamino- oder mono- oder disubstituierte Aminogruppe, oder eine Gruppe der Formel

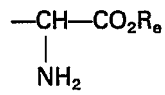

in der $R_e$ Wasserstoff, Methyl oder Äthyl ist und m eine ganze Zahl von 1 bis 3 ist; ein Phenyl-, Phenoxy- oder Phenylthioring, unsubstituiert oder mono- oder polysubstituiert in den m-, o- und p-Stellungen; eine Gruppe der Formel —$(CH_2)_m$—SCO—$(CH_2)_nP_3$ in der m und n die vorstehend angegebenen Bedeutungen haben und $P_3$ eine lineare oder verzweigte $C_1$—$C_7$-Niederalkylkette, ein $C_3$—$C_6$-Cycloalkylrest, eine disubstituierte Aminogruppe, ein Phenyl- oder Phenoxyring, gegebenenfalls mono- oder polysubstituiert in den o-, m- und p-Stellungen, eine Alkenylkette der Formel

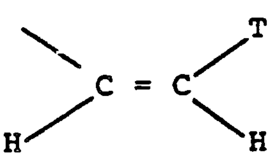

in der T zusätzlich zu den vorstehend angegebenen Bedeutungen auch Wasserstoff sein kann; der Begriff "monosubstituierte Aminogruppe" innerhalb seiner Bedeutungen eine durch einen linearen oder verzweigten $C_1$—$C_6$-Alkylrest oder durch Gruppen der Formel:

$$-CH_2-CH_2-O-CH_2-CH_3, \quad -CH_2-CH_2-O-CH_2-CH_2-OH,$$

$-CH_2-CH_2-NH-CH_2-CH_3$, $-CH_2-CH_2-NH-CH_2-CH_2-OH$ oder

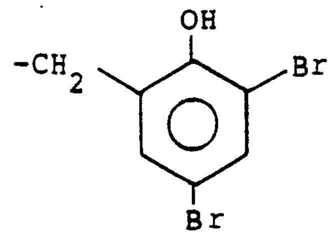

substituierte Aminogruppe umfaßt;
die Substituenten einer disubstituierten Aminogruppe gemäß der Erfindung lineare oder verzweigte $C_1$—$C_6$-Alkylreste sein können oder zusammengenommen einen ungesättigten oder gesättigten Stickstoff-Ring darstellen, wie Morpholin-1-yl, Pyrrolidin-1-yl, Piperidin-1-yl, 4-Methyl-piperazin-1-yl, Thiomorpholin-1-yl, 4-Äthylpiperazin-1-yl, 4-(2'-Fluorphenyl)-piperazin-1-yl, Imidazol-1-yl, 3-Pyridyl, 4-Pyridyl;

und schließlich der Begriff "mono- oder polysubstituierte Phenylgruppe" gemäß der Erfindung Phenylgruppen bedeutet, die durch ein Fluoratom in para-Stellung, durch Chloratome in den meta- und/oder para-Stellungen oder durch ein $CF_3$ in den meta-Stellungen substituiert sind, oder eine Phenylgruppe der Formel

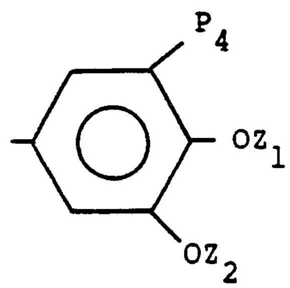

in der $Z_1$ H oder $COCH_3$ ist und $Z_2$ H, $CH_3$ oder $COCH_3$ bedeutet und $P_4$ in der Gruppe ausgewählt ist, die aus Wasserstoff, Aminomethyl, $C_1$—$C_2$-Acylaminomethyl oder mono- oder disubstituierte Aminomethylgruppen gemäß vorstehen der Definition besteht,
dadurch gekennzeichnet, daß eine Verbindung der Formel II

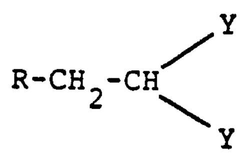

(II)

in der R wie vorstehend definiert ist und sowohl Y als auch Y' $C_1$—$C_2$-Niederalkoxyreste sind oder zusammengenommen eine Carbonylgruppe bilden, mit einem Aminoalkanthiol der Formel III

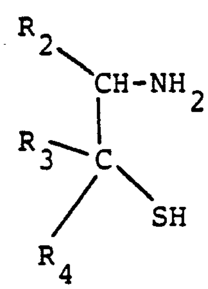

(III)

in der $R_2$, $R_3$ und $R_4$ wie vorstehend definiert sind, umgesetzt wird, und das die erhaltenen Verbindungen der Formel (Ia)

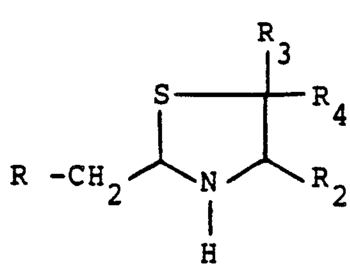

(Ia)

in der R, $R_2$, $R_3$ und $R_4$ wie vorstehend definiert sind, dann gegebenenfalls mit Bernsteinsäure- oder Glutarsäureanhydrid oder mit einer Verbindung der Formel umgesetzt werden

$Q-(CH_2)_n-COZ$

(IVa)

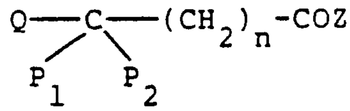

(IVb)

$P_5-SO_2-Hal$

(IVc)

wobei:

Q, $P_1$, $P_2$ und n wie vorstehend definiert sind;

$P_5$ einen $C_1$—$C_2$-Alkylrest oder einen unsubstituierten oder mono- oder polysubstituierten Phenylring bedeutet;

Hal ein Halogenatom ist und Z eine aktivierende Gruppe der Carboxyeinheit, wie Halogen, Azid, aktivierte Ester oder Anhydride, bedeutet.

2. Verfahren zur Herstellung von Verbindungen der Formel I, in denen $R_1$ eine Gruppe der Formel

$-CH=CH-T$ oder 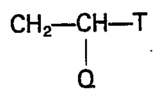

bedeutet, wobei T und Q wie vorstehend definiert sind, dadurch gekennzeichnet, daß eine Verbindung der Formel Ie

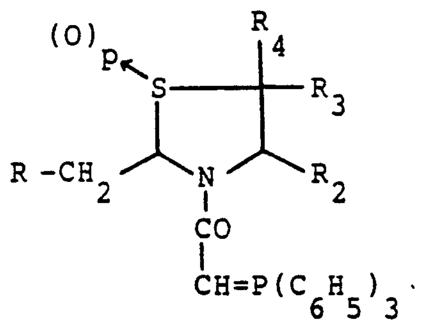

(Ie)

in der R, $R_3$, $R_4$, $R_2$ und p wie vorstehend definiert sind, mit einem Aldehyd der Formel V

$$T-CHO \qquad (V)$$

in der T wie vorstehend definiert ist, umgesetzt wird, und daß die so erhaltenen Verbindungen gegebenenfalls einer Michael-Reaktion mit nucleophilen Verbindungen unterzogen werden, die in der Gruppe der Amine der Formel $H_2N-(CH_2)_m-T_1$; mono- oder disubstituierten Amine oder Thiole der Formel $HS-(CH_2)_n-T_1$, oder gegebenenfalls mono- oder disubstituierten Thiophenole ausgewählt sind, wobei m, n und $T_1$ wie vorstehend definiert sind, unterzogen werden.

3. Verfahren zur Herstellung von Verbindungen der Formel I, in der $R_1$ eine Gruppe der Formel

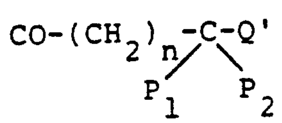

in der n, $P_1$ und $P_2$ wie vorstehend definiert sind und Q' eine SH—, eine Alkylthioäthergruppe oder eine Gruppe der Formel $P_3-(CH_2)_n-COS-$ bedeuten,
dadurch gekennzeichnet, daß eine Verbindung der Formel Id

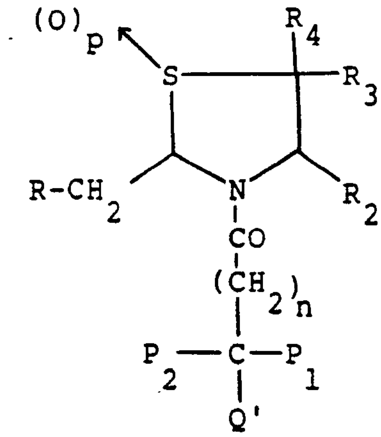

(Id)

in der R, $R_2$, $R_3$, $R_4$ und p wie vorstehend definiert sind, mindestens einer der Reste $P_1$ und $P_2$ Wasserstoff ist und der andere Wasserstoff, Methyl oder Phenyl bedeutet, Q′ ein Halogenatom darstellt und n vorzugsweise 0 ist, mit einem Thiocarbonsäuresalz der Formel

$$P_3—(CH_2)_n—COS^{(-)} \quad Base^{(+)}$$

in der $P_3$ und n wie vorstehend definiert sind und die Base ein Alkali- oder Erdalkalimetall, eine niedere Trialkylammonium- oder Phenyldialkylammoniumgruppe bedeutet, umgesetzt wird, und daß die so erhaltenen Verbindungen mit Ammoniak umgesetzt werden, wobei Q=SH erhalten wird, das dann gegebenenfalls mit einem Alkylhalogenid in Gegenwart einer Base alkyliert werden kann.

**Revendications pour les Etats contractants: BE CH DE FR GB IT LI LU NL SE**

1. Composé de formule (I):

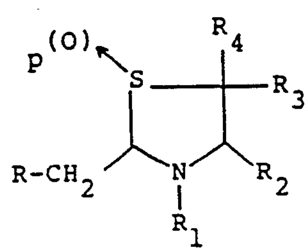

(I)

dans laquelle:

R représente diméthyl-1,3 xanthinyle-7 ou diméthyl-3,7 xanthinyle-1;

$R_2$ représente hydrogène, un groupe carboxy ou, lorsque p = 0 et $R_1 = R_3 = R_4 = H$, un groupe carboxy estérifié par des alcools en $C_1—C_5$;

$R_3$ et $R_4$ représentent tous deux hydrogène ou méthyle;

p vaut zéro ou 1;

$R_1$ représente: hydrogène; un groupe alkyle sulfonyle en $C_1—C_2$; un groupe phényl sulfonyle non-substitué ou mono- ou polysubstitué; ou un groupe acyle de formule

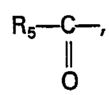

où $R_5$ représente hydrogène,

$-(CH_2)_n-Q$ et 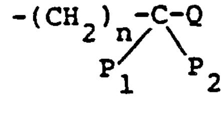

où:

n vaut 0 ou un nombre entier de 1 à 7;

$P_1$, $R_2$ représentent tous deux hydrogène ou l'un d'entre eux représente hydrogène et l'autre représente alkyle inférieur en $C_1—C_4$ ou phényle; et

Q est choisi dans le groupe constitué par:

hydrogène;

un groupe alkyle ramifié en $C_3—C_4$;

un groupe cycloalkyle en $C_3$—$C_7$;
un groupe carboxy libre ou $COOC_2H_5$ lorsque $R_2 = R_3 = R_4 = H$;
un atome d'halogène;
SH;
—$NH_2$;
un groupe amino mono- ou disubstitué, tert.-butoxy carbonylamino ou acylamino en $C_1$—$C_2$;

une chaîne éther —O—T ou thioéther S—T, où T représente un cycle phényle non-substitué ou mono- ou polysubstitué ou un groupe de formule $(CH_2)_m$—$T_1$, où $T_1$ est choisi dans le groupe constitué par H, OH, $OCH_3$, $OC_2H_5$, $HOCH_2$—$CH_2$—, un groupe carboxy libre ou $COOC_2H_5$ lorsque $R_2 = R_3 = R_4 = H$, $NH_2$, un groupe acylamino en $C_1$—$C_2$ ou amino mono- ou disubstitué, ou un groupe de formule

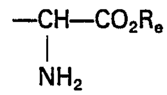

où $R_e$ représente hydrogène, méthyle ou éthyle et m représente un nombre entier de 1 à 3;

un cycle phényle, phénoxy ou phénylthio, non-substitué ou mono- ou polysubstitué dans les positions m, o et p;

un groupe de formule —$(CH_2)_m$—SCO—$(CH_2)_nP_3$, où m et n ont les significations définies ci-dessus et $P_3$ représente un chaîne alkyle inférieur en $C_1$—$C_7$, linéaire ou ramifiée, un groupe cycloalkyle en $C_3$—$C_6$, un groupe amino disubstitué, un cycle phényle ou phénoxy, le cas échéant mono- ou polysubstitué dans les positions o, m et p;

une chaîne alcényle de formule:

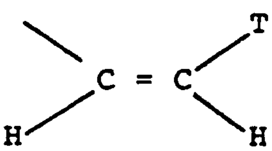

où T, en plus des significations définies ci-dessus, peut représenter également hydrogène;

l'expression ≪groupe amino monosubstitué≫ comprenant, dans ses significations, un groupe amino substitué par un groupe alkyle en $C_1$—$C_6$, linéaire ou ramifié, ou par des groupes ayant les formules:

$-CH_2-CH_2-O-CH_2-CH_3$, $-CH_2-CH_2-O-CH_2-CH_2-OH$,

$-CH_2-CH_2-NH-CH_2-CH_3$, $-CH_2-CH_2-NH-CH_2-CH_2-OH$ ou

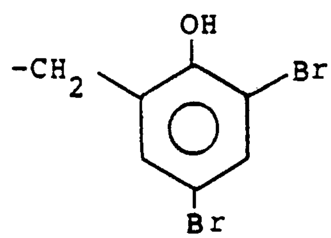

les substituants d'un groupe amino disubstitué selon l'invention pouvant être des groupes alkyle en $C_1$—$C_6$, linéaires ou ramifiés, ou, pris ensemble, représentant un cycle azoté, insaturé ou saturé, tel que morpholinyle-1, pyrrolidinyle-1, pipéridinyle-1, méthyl-4 pipérazinyle-1, thiomorpholinyle-1, éthyle-4 pipérazinyle-1, (fluoro-2' phényl)-4 pipérazinyle-1; imidazolyle-1, pyridyle-3, pyridyle-4;

enfin, l'expression "phényle mono- ou polysubstitue", selon l'invention, signifiant des groupes phényle qui sont substitués par un atome de fluor dans la position para, par des atomes de chlore dans les positions méta et/ou para ou par un $CF_3$ dans les positions méta, ou un groupe phényle de formule:

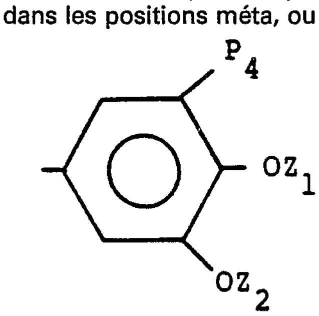

dans laquelle:

$Z_1$ représente H ou $COCH_3$; et

$Z_2$ représente H, $CH_3$ ou $COCH_3$; et

$P_4$ est choisi dans le groupe constitué par hydrogène, aminométhyle, acyl en $C_1$—$C_2$-aminométhyle ou un groupe aminométhyle mono- ou disubstitué, tel que défini ci-dessus.

2. Composés de formule (I) dans laquelle $R_2$, $R_3$ et $R_4$ représentent hydrogène et $R_1$ représente un groupe acyle de formule $R_5CO$, où $R_5$ a les significations définies ci-dessus.

3. Composés de formule (I) dans laquelle $R_2$, $R_3$ et $R_4$ représentent hydrogène et $R_1$ représente un groupe cinnamoyle substitué ou non-substitué.

4. Composé choisi dans le groupe constitué par:

l'acide [(diméthyl-1',3' xanthinyl-7')méthyl]-2 thiazolidine carboxylique-4 et ses esters méthylique et tert.-butylique;

l'acide [(diméthyl-3',7' xanthinyl-1')méthyl]-2 thiazolidine carboxylique-4 et ses esters méthylique, éthylique et tert.-butylique;

la [(diméthyl-1',3' xanthinyl-7')méthyl]-2 thiazolidine;

la [(diméthyl-3',7' xanthinyl-1')méthyl]-2 thiazolidine;

la [(diméthyl-1',3' xanthinyl-7')méthyl)-2 [(imidazolyl-1')acétyl]-3 thiazolidine;

la [(diméthyl-3',7' xanthinyl-1')méthyl]-2 [(imidazolyl-1')acétyl]-3 thiazolidine;

la [(diméthyl-1',3' xanthinyl-7')méthyl]-2 [(dihydroxy-3',4')cinnamoyl]-3 thiazolidine;

la [(diméthyl-3',7' xanthinyl-1')méthyl]-2 [(dihydroxy-3',4')cinnamoyl]-3 thiazolidine;

la [(diméthyl-1',3' xanthinyl-7')méthyl]-2 (méthoxy-3' hydroxy-4')cinnamoyl -3 thiazolidine;

la [(diméthyl-3',7' xanthinyl-1')méthyl]-2 (méthoxy-3' hydroxy-4')cinnamoyl -3 thiazolidine;

la [(diméthyl-3',7' xanthinyl-1')méthyl]-2 [(hydroxyéthyl-4'' pipérazinyl-1'')-3' propionyl]-3 thiazolidine;

la [(diméthyl-1',3' xanthinyl-7')méthyl]-2 (morpholinométhyl-3' hydroxy-4' méthoxy-3' cinnamoyl)-3 thiazolidine;

la [(diméthyl-1',3' xanthinyl-7')méthyl]-2 (pyrrolidylméthyl-3' hydroxy-4' méthoxy-3' cinnamoyl)-3 thiazolidine;

la [(diméthyl-3',7' xanthinyl-1')méthyl]-2 (pyrrolidinylméthyl-3' hydroxy-4' méthoxy-3' cinnamoyl)-3 thiazolidine;

la [(diméthyl-1',3' xanthinyl-7')méthyl]-2 [thia-3' carboxy-5' acétylamino-5' pentanoyl]-3 thiazolidine;

la [(diméthyl-1',3' xanthinyl-7')méthyl]-2 [thia-4' carboxy-6' acétylamino-6' hexanoyl]-3 thiazolidine;

la [(diméthyl-1',3' xanthinyl-7')méthyl]-2 [(éthoxy-2''éthyloxy)-3' acétyl]-3 thiazolidine;

la [(diméthyl-1',3' xanthinyl-7')méthyl]-2 [(imidazolyl-1')-3' propionyl]-3 thiazolidine;

la [(diméthyl-1',3' xanthinyl-7')méthyl]-2 glycinyl-3 thiazolidine;

la [(diméthyl-3',7' xanthinyl-1')méthyl]-2 glycinyl-3 thiazolidine;

la [(diméthyl-1',3' xanthinyl-7')méthyl]-2 (N-acétyl)glycinyl-3 thiazolidine;

la [(diméthyl-1',3' xanthinyl-7')méthyl]-2 [(hydroxy-2''éthylamino)éthylamino-3' propionyl]-3 thiazolidine;

la [(diméthyl-3',7' xanthinyl-1')méthyl]-2 [(hydroxy-2''éthylamino)éthylamino-3' propionyl]-3 thiazolidine;

la [(diméthyl-1',3' xanthinyl-7')méthyl]-2 [(hydroxyéthyl-4'' pipérazinyl-1'')-3' propionyl]-3 thiazolidine.

5. Procédé de fabrication de composés de formule (I), caractérisé par le fait qu'on fait réagir un composé de formule (II):

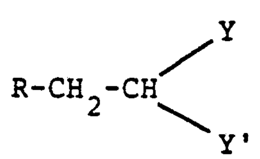

(II)

dans laquelle:

R est tel que défini ci-dessus; et

Y et Y' représentent tous deux alcoxy inférieur en $C_1$—$C_2$ ou, pris ensemble, forment un groupe carboxyle, avec un aminoalcanethiol de formule (III):

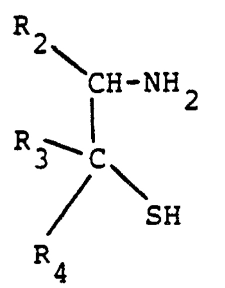

(III)

dans laquelle $R_2$, $R_3$ et $R_4$ sont tels que définis ci-dessus, et que, de façon facultative, ou fait ensuite réagir les composés obtenus de formule (Ia):

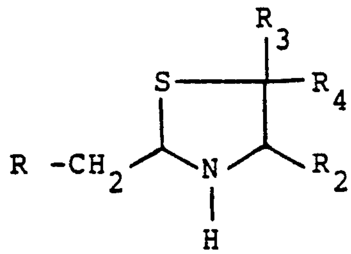

(Ia)

dans laquelle R, $R_2$, $R_3$, $R_4$ sont tels que définis ci-dessus, avec l'anhydride succinique ou glutarique ou un composé de formule:

$Q-(CH_2)_n-COZ$ (IVa)

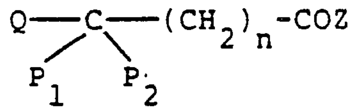

(IVb)

$P_5-SO_2-Hal$ (IVc)

où:

Q, $P_1$, $P_2$ et n sont tels que définis ci-dessus;

$P_5$ représente un groupe alkyle en $C_1$—$C_2$ ou un cycle phényle non-substitué ou mono- ou polysubstitué;

Hal représente un atome d'halogène; et

Z représente un group activant de la fraction carboxy, tel qu'halogène, azide, esters activés ou anhydrides.

6. Procédé de fabrication de composés de formule (I) dans laquelle $R_1$ représente un groupe de formule

$$—CH=CH—T \text{ ou } CH_2—\underset{\underset{Q}{|}}{CH}—T$$

où T et Q sont tels que définis ci-dessus, caractérisé par le fait qu'on fait réagir un composé de formule (Ie):

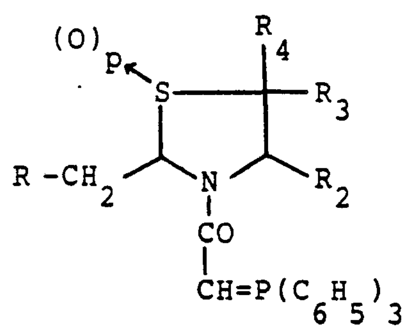

(Ie)

où R, $R_3$, $R_4$, $R_2$ et p sont tels que définis ci-dessus, avec un aldéhyde de formule (V):

$$T—CHO \qquad (V)$$

où T est tel que déini ci-dessus et que, de façon facultative, on soumet les composés ainsi obtenus à une réaction de Michael avec des composés nucléophiles choisis dans le groupe des amines de formule $H_2N—(CH_2)_m—T_1$; amines mono- ou disubstituées ou thiols de formule $HS—(CH_2)_n—T_1$, thiophénols facultativement mono ou disubstitués, m, n et $T_1$ étant tels que définis ci-dessus.

7. Procédé de fabrication de composés de formule (I), dans laquelle $R_1$ représente un groupe de formule:

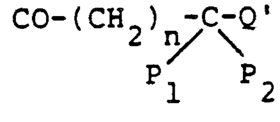

dans laquelle:

n, $P_1$ et $P_2$ sont tels que définis ci-dessus; et

Q' représente un groupe SH, un groupe alkylthioéther ou un groupe de formule:

$$P_3—(CH_2)_n—COS—$$

caractérisé par le fait qu'on fait réagir un composé de formule (Id):

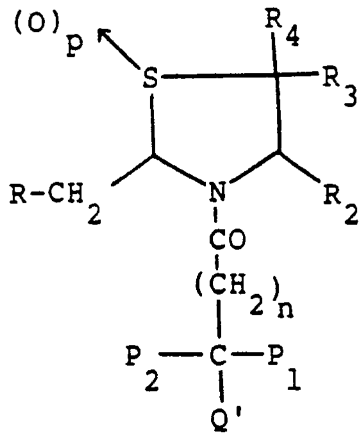

(Id)

dans laquelle:

R, $R_2$, $R_3$, $R_4$, p sont tels que définis ci-dessus;

au moins l'un parmi $P_1$ et $P_2$ représente hydrogène, et l'autre représente hydrogène, méthyle ou phényle;

Q' représente un atome d'halogène; et

n vaut de préférence zéro,

avec un sel d'acide thiocarboxylique de formule:

$$P_3—(CH_2)_n—COS^{(-)}\ Base^{(+)}$$

dans laquelle:

$P_3$ et n sont tels que définis ci-dessus; et

la base est un métal alcalin ou alcalino-terreux, trialkylammonium inférieur ou phényldialkylammonium, et qu'on fait réagir les composés ainsi obtenus avec l'ammoniac pour donner Q = SH, lequel peut, de façon facultative, être alkylé avec un halogénure d'alkyle en présence d'une base.

8. Compositions pharmaceutiques ayant une activité régulatrice du mucus, bronchodilatatrice, antitussive, contenant une quantité thérapeutiquement efficace d'un composé tel que revendiqué à l'une des revendications 1—4, en mélange avec des supports ou excipients non-toxiques.

**Revendications pour l'Etat contractant: AT**

1. Procédé de fabrication de composés de formule (I):

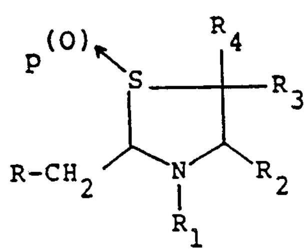

(I)

dans laquelle:

R représente diméthyl-1,3 xanthinyle-7 ou diméthyl-3,7 xanthinyle-1;

$R_2$ représente hydrogène, un groupe carboxy ou, lorsque p = 0 et $R_1 = R_3 = R_4$ = H, un groupe carboxy estérifié par des alcools en $C_1—C_5$;

$R_3$ et $R_4$ représentent tous deux hydrogène ou méthyle;

p vaut zéro ou 1;

$R_1$ représente: hydrogène; un groupe alkyle sulfonyle en $C_1—C_2$; un groupe phényl sulfonyle non-substitué ou mono- ou polysubstitué; ou un groupe acyle de formule

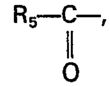

où $R_5$ représente hydrogène,

$-(CH_2)_n-Q$ et 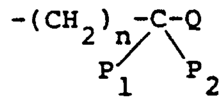

où:

n vaut 0 ou un nombre entier de 1 à 7;

$P_1$, $P_2$ représentent tous deux hydrogène ou l'un d'entre eux représente hydrogène et l'autre représente alkyle inférieure en $C_1$—$C_4$ ou phényle; et

Q est choisi dans le groupe constitué par:

hydrogène;

un groupe alkyle ramifié en $C_3$—$C_4$;

un groupe cycloalkyle en $C_3$—$C_7$;

un groupe carboxy libre ou $COOC_2H_5$ lorsque $R_2 = R_3 = R_4 = H$;

un atome d'halogène;

SH;

—$NH_2$;

un groupe amino mono- ou disubstitué, tert.-butoxy carbonylamino ou acylamino en $C_1$—$C_2$;

une chaîne éther —O—T ou thioéther S—T, où T représente un cycle phényle non-substitué ou mono- ou polysubstitué ou un groupe de formule $(CH_2)_m$—$T_1$, où $T_1$ est choisi dans le groupe constitué par H, OH, $OCH_3$, $OC_2H_5$, $HOCH_2$—$CH_2$—, un groupe carboxy libre ou $COOC_2H_5$ lorsque $R_2 = R_3 = R_4 = H$, $NH_2$, un groupe acylamino en $C_1$—$C_2$ ou amino mono- ou disubstitué, ou un groupe de formule

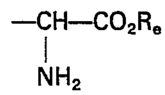

où $R_e$ représente hydrogène, méthyle ou éthyle et m représente un nombre entier de 1 à 3;

un cycle phényle, phénoxy ou phénylthio, non-substitué ou mono- ou polysubstitué dans les positions m, o et p;

un groupe de formule —$(CH_2)_m$—SCO—$(CH_2)_nP_3$, où m et n ont les significations définies ci-dessus et $P_3$ représente un chaîne alkyle inférieur en $C_1$—$C_7$, linéaire ou ramifiée, un groupe cycloalkyle en $C_3$—$C_6$, un groupe amino disubstitué, un cycle phényle ou phénoxy, le cas échéant mono- ou polysubstitué dans les positions o, m et p;

une chaîne alcényle de formule:

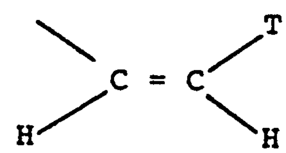

où T, en plus des significations définies ci-dessus, peut représenter également hydrogène;

l'expression «groupe amino monosubstitué» comprenant, dans ses significations, un groupe amino substitué par un groupe alkyle en $C_1$—$C_6$, linéaire ou ramifié, ou par des groupes ayant les formules:

$-CH_2-CH_2-O-CH_2-CH_3$, $-CH_2-CH_2-O-CH_2-CH_2-OH$,

$-CH_2-CH_2-NH-CH_2-CH_3$, $-CH_2-CH_2-NH-CH_2-CH_2-OH$ ou

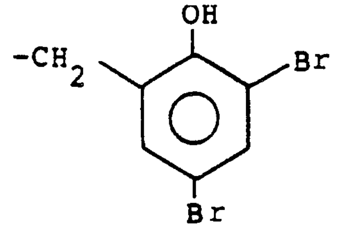

les substituents d'un groupe amino disubstitué selon l'invention pouvant être des groupes alkyle en $C_1$—$C_6$, linéaires ou ramifiés, ou, pris ensemble, représentant un cycle azoté, insaturé ou saturé, tel que

morpholinyle-1, pyrrolidinyle-1, pipéridinyle-1, méthyl-4 pipérazinyle-1, thiomorpholinyle-1, éthyle-4 pipérazinyle-1, (fluoro-2' phényl)-4 pipérazinyle-1; imidazolyle-1, pyridyle-3, pyridyle-4;

enfin, l'expression "phényle mono- ou polysubstitué", selon l'invention, signifiant des groupes phényle qui sont substitués par un atome de fluor dans la position para, par des atomes de chlore dans les positions méta et/ou para ou par un $CF_3$ dans les positions méta, ou un groupe phényle de formule:

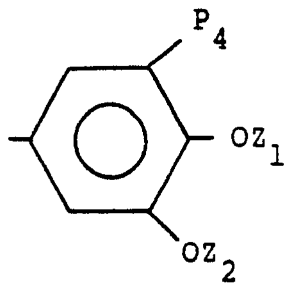

dans laquelle:

$Z_1$ représente H ou $COCH_3$; et

$Z_2$ représente H, $CH_3$ ou $COCH_3$; et

$P_4$ est choisi dans le groupe constitué par hydrogène, aminométhyle, acyl en $C_1$—$C_2$-aminométhyle ou un groupe aminométhyle mono- ou disubstitué, tel que défini ci-dessus, caractérisé par le fait qu'on fait réagir un composé de formule (II):

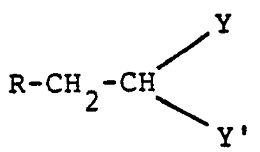

(II)

dans laquelle:

R est tel que défini ci-dessus; et

Y et Y' représentent tous deux alcoxy inférieur en $C_1$—$C_2$ ou, pris ensemble, forment un groupe carboxyle, avec un aminoalcanethiol de formule (III):

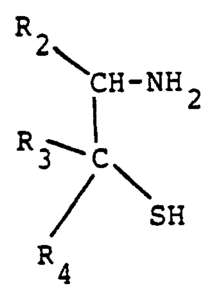

(III)

dans laquelle $R_2$, $R_3$ et $R_4$ sont tels que définis ci-dessus, et que, de façon facultative, ou fait ensuite réagir les composés obtenus de formule (Ia):

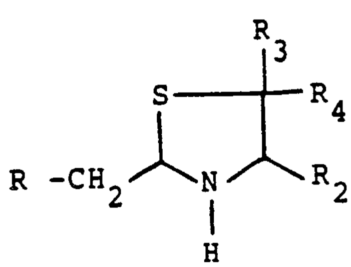

(Ia)

dans laquelle R, $R_2$, $R_3$, $R_4$ sont tels que définis ci-dessus, avec l'anhydride succinique ou glutarique ou un composé de formule:

$Q-(CH_2)_n-COZ$ 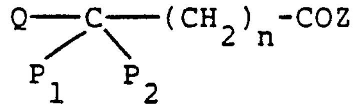 $P_5-SO_2-Hal$

(IVa) (IVb) (IVc)

où:

Q, $P_1$, $P_2$ et n sont tels que définis ci-dessus;

$P_5$ représente un groupe alkyle en $C_1$—$C_2$ ou un cycle phényle non-substitué ou mono- ou polysubstitué;

Hal représente un atome d'halogène; et

Z représente un group activant de la fraction carboxy, tel qu'halogène, azide, esters activés ou anhydrides.

2. Procédé de fabrication de composés de formule (I) dans laquelle $R_1$ représente un groupe de formule

—CH=CH—T ou 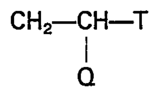

où T et Q sont tels que définis ci-dessus, caractérisé par le fait qu'on fait réagir un composé de formule (Ie):

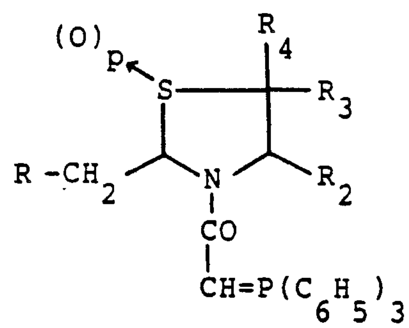

(Ie)

où R, $R_3$, $R_4$, $R_2$ et p- sont tels que définis ci-dessus, avec un aldéhyde de formule (V):

$$T—CHO \qquad (V)$$

où T est tel que défini ci-dessus et que, de façon facultative, on soumet les composés ainsi obtenus à une réaction de Michael avec des composés nucléophiles choisis dans le groupe des amines de formule $H_2N—(CH_2)_m—T_1$; amines mono- ou disubstituées ou thiols de formule $HS—(CH_2)_n—T_1$, thiophénols facultativement mono- ou disubstitués, m, n et $T_1$ étant tels que définis ci-dessus.

3. Procédé de fabrication de composés de formule (I), dans laquelle $R_1$ représente un groupe de formule:

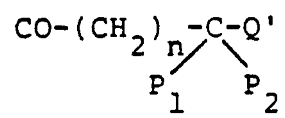

dans laquelle:

n, $P_1$ et $P_2$ sont tels que définis ci-dessus; et

Q' représente un groupe SH, un groupe alkylthioéther ou un groupe de formule:

$$P_3—(CH_2)_n—COS—$$

caractérisé par le fait qu'on fait réagir un composé de formule (Id):

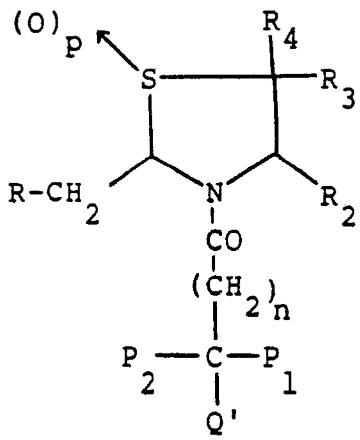

(Id)

dans laquelle:

R, $R_2$, $R_3$, $R_4$, p sont tels que définis ci-dessus;

au moins l'un parmi $P_1$ et $P_2$ représente hydrogène, et l'autre représente hydrogène, méthyle ou phényle;

Q' représente un atome d'halogène; et

n vaut de préférence zéro,

avec un sel d'acide thiocarboxylique de formule:

$$P_3—(CH_2)_n—COS^{(-)}\ Base^{(+)}$$

dans laquelle:

$P_3$ et n sont tels que définis ci-dessus; et

la base est un métal alcalin ou alcalino-terreux, trialkylammonium inférieur ou phényldialkylammonium, et qu'on fait réagir les composés ainsi obtenus avec l'ammoniac pour donner Q = SH, lequel peut, de façon facultative, être alkylé avec un halogénure d'alkyle en présence d'une base.